Europäisches Patentamt

(19) European Patent Office

Office européen des brevets

(11) Publication number: **0 084 796**
**B1**

(12) **EUROPEAN PATENT SPECIFICATION**

(45) Date of publication of patent specification: **02.05.90**

(21) Application number: **83100175.5**

(22) Date of filing: **11.01.83**

(51) Int. Cl.⁵: **C 12 N 15/00, C 12 Q 1/68**

(54) HLA typing method and cDNA probes used therein.

(30) Priority: **22.01.82 US 341902**

(43) Date of publication of application:
**03.08.83 Bulletin 83/31**

(45) Publication of the grant of the patent:
**02.05.90 Bulletin 90/18**

(84) Designated Contracting States:
**AT BE CH DE FR GB IT LI LU NL SE**

(56) References cited:
**WO-A-82/02060**

**PROCEEDINGS NATIONAL ACADEMY OF SCIENCE USA, vol. 79, January 1982, pages 545-49; H.S.LEE et al.: "cDNA clones coding for the heavy chain of human HLA-DR antigen".**

**CHEMICAL ABSTRACTS, vol. 94, no. 17, 27th April 1981, page 579, no. 137559z, Columbus Ohio (USA);A.K.SOOD et al.: " Isolated and partial nucleotide sequence of a cDNA clone for human histocompatibility antigen HLA-B by use of an oligodeoxynucleotide primer".**

(73) Proprietor: **CETUS CORPORATION**
**1400 Fifty-Third Street**
**Emeryville California 94608 (US)**

(72) Inventor: **Erlich, Henry A.**
**3936 Rhoda Avenue**
**Oakland California 94602 (US)**

(74) Representative: **Wuesthoff, Franz, Dr.-Ing. et al**
**Wuesthoff & Wuesthoff Patent- und**
**Rechtsanwälte Schweigerstrasse 2**
**D-8000 München 90 (DE)**

(56) References cited:
**CHEMICAL ABSTRACTS, vol. 95, no. 15, October 1981, page 477, no. 130574n, Columbus Ohio (USA);P.ROTWEIN et al.: " Polymorphism in the 5' - flanking region of the human insulin gene and its possible relation to type 2 diabetes".**

**PROCEEDINGS NATIONAL ACADEMY OF SCIENCE USA, vol. 75, no. 11, November 1978, pages 5631-5635;;Y.W.KAN et al.: "Polymorphism of DNA sequence adjacent to human beta-globin structural gene: Relationship to sickle mutation".**

Courier Press, Leamington Spa, England.

# EP 0 084 796 B1

(56) References cited:
PROCEEDINGS NATIONAL ACADEMY OF
SCIENCE USA, vol. 77, no. 10, October 1980,
pages 6081-6085;H. PLOEGH et al.: "Molecular
cloning of a human histocompatibility antigen
cDNA fragment".

NATURE, vol. 290, April 1981, pages 521-523,
(UK);79, January 1982, pages 545-
49;B.R.JORDAN et al.: "Human HLA gene
segment isolated by hybridization with mouse
H-2 cDNA probes".

NATURE, vol. 291, 25th. June 1981, pages
673-675, (UK); B.CAMI et al.: "Multiple
sequences related to classical
histocompatibility antigens in the mouse
genome".

## Description

Technical Field

The invention is in the fields of genetic engineering and human genotyping. More specifically the invention concerns methods for HLA typing based on HLA DNA restriction fragment length polymorphism and to novel complementary DNA (cDNA) probes that are used in such methods.

Background Art

The major histocompatibility complex (MHC) of humans is a cluster of genes occupying a region located on the sixth chromosome. This complex, denoted HLA (Human Leukocyte Antigen), has been divided into five major gene loci, which according to World Health Organization nomenclature are designated HLA-A, HLA-B, HLA-C, HLA-D, and HLA-DR. The A, B, and C loci are single gene loci. The D and DR loci are multi-gene loci. The A, B, and C loci encode the classical transplantation antigens, whereas the D and DR loci encode products that control immune responsiveness. More recent definitions divide the gene products of the HLA loci into three classes (I, II, and III) based on structure and function *(Nature* (1982) 297:692-632). Class I encompasses the products of the HLA-A, HLA-B, and HLA-C loci and the Qa/TL region. The products of the HLA-D and HLA-DR related genes fall in Class II. The Class II antigens are believed to be heterodimers composed of an α (~34,000 daltons) glycopeptide and a β (~29,000 daltons) glycopeptide. The number of loci and the gene order of Class II are tentative. Class II currently includes loci designated DRα, DRβ, DSβ, DC(α or β), and SB. It is likely that future investigation will reveal additional Class II loci. The third class, Class III, includes components of complement. As used herein, the term "HLA" is intended to include the above described loci as well as loci that are closely linked thereto.

The products encoded by the HLA loci are currently typed serologically or by mixed lymphocyte culture methods. Such typing is used in paternity determinations, transplant and transfusion compatibility testing, blood component therapy, anthropological studies and in disease association correlation to diagnose diseases or predict susceptibility to diseases. The major drawbacks to such HLA typing, particularly of the Class II loci, are the complexity of the sera and the lack of widespread availability of standard sera necessary to conduct the tests. Also, since serological typing is based on reactions of sera with the HLA gene products it may not be useful for fetal HLA typing in the early stages of pregnancy when those products have not yet been expressed. Further, the lymphocytotoxicity test often gives results that do not provide an adequate basis for recognizing Class II locus specificities.

It is well known that there is extensive polymorphism in the DNA of the human population. Recent work has also found polymorphism in the restriction endonuclease digests of human DNA. Restriction endonucleases recognize specific nucleotide sequences in DNA and catalyze endonucleolytic cleavages, yielding DNA fragments of defined length. Differences among individuals in the lengths of a particular restriction fragment are called "restriction length fragment polymorphisms" (RFLPs). Kan and Zozy, *PNAS* (1978) 75:5361-5635 report RFLPs produced by *Hpa*I cleavage of human β-globin genes and an apparent association between a 13.0 kb variant of the normal 7.6 kb fragment and sickle hemoglobin mutation. These RFLPs were detected by comparing Southern blots of *Hpa*I restricted cellular DNA from individuals with normal hemoglobin, sickle cell trait, and sickle cell anemia probed with a radiolabeled β-globin cDNA probe.

Botstein, et al, *Am J Human Genet* (1980) 32:314—331, have proposed using RFLPs as genetic markers to construct a genetic linkage map of the human genome. Their proposal contemplates identifying polymorphic loci by Southern blotting using restricted DNA and single strand cDNA probes, testing the loci for linkage relationships in human pedigrees, and arranging the loci into linkage groups to form a genetic map.

Sood, et al, *PNAS* (1981) 78:616—620 (also PCT application 8202060 published 24 June 1982), describe the isolation of cDNA clones for HLA—B antigens. These clones were prepared by synthesizing cDNA from an mRNA mix containing mRNA coding for the desired HLA antigen, inserting the cDNA into a vector, transforming a bacterial host and isolating transformant clones that contain the desired DNA segment by probing with an oligonucleotide probe that is specific for the desired DNA sequence. Ploegh, et al *PNAS* (1980) 77:6081—6085 have also reported cloning a cDNA probe for an HLA gene sequence.

Disclosure of the Invention

One aspect of the invention is an HLA typing method based on HLA restriction length polymorphism characterized by the following steps:

(a) digesting genomic HLA DNA from an individual with a restriction endonuclease that produces a polymorphic digestion pattern of a Class II, HLA, DNA locus;

(b) subjecting the digest of (a) to genomic blotting using a labeled cDNA hybridization probe that is complementary to a Class II HLA DNA sequence involved in the polymorphism; and

(c) comparing the genomic blotting pattern obtained in (b) with a standard genomic blotting pattern for said HLA DNA sequence obtained using said restriction endonuclease and the same labeled cDNA hybridization probe or one having the same specificity.

As applied to paternity testing the method involves:

(a) digesting genomic HLA DNA of the mother of the individual, the suspected father of the individual, and the individual with a restriction endonuclease that produces a polymorphic digestion pattern of a Class II HLA DNA locus.

(b) subjecting each of the digests of (a) to genomic blotting using a labeled cDNA hybridization probe that is complementary to an HLA DNA sequence involved in the polymorphism; and

(c) comparing the genomic blotting patterns obtained in (b) to determine correspondence between the individual's pattern and the mother's and suspected father's pattern and thereby determining whether the suspected father is the actual father of the individual.

As applied to determining whether an individual has or is susceptible to a disease the method is characterized by the following steps:

(a) digesting genomic HLA DNA from the individual with a restriction endonuclease that produces a polymorphic digestion pattern of a Class II HLA locus that is associated with the disease;

(b) subjecting the digest of (a) to genomic blotting using a labeled cDNA probe that is complementary to the DNA sequence of said HLA locus;

(c) comparing the genomic blotting pattern obtained in (b) with a standard pattern of an individual having said disease obtained using said restriction endonuclease and the same labeled cDNA probe or one having the same specificity.

As applied to determining transplant or transfusion compatibility the method is characterized by the following steps:

(a) digesting genomic HLA DNA of the transplant or transfusion donor and the transplant or transfusion host with a restriction endonuclease that produces a polymorphic digestion pattern of a Class.II HLA DNA locus;

(b) subjecting each of the digests of (a) to genomic blotting using a labeled cDNA probe that is complementary to a Class II HLA DNA sequence involved in the polymorphism; and

(c) comparing the genomic blotting patterns obtained in (b) to determine correspondence therebetween and thereby determining whether the transplant or transfusion donor's HLA is compatible with the transplant or transfusion host's HLA.

Another aspect of the invention is an HLA cDNA probe characterized in that the probe is specific to a single Class II HLA locus and comprises a labeled DNA sequence that is complementary to the DNA sequence at said locus.

Another aspect of the invention is an HLA cDNA sequence characterized in that the sequence is complementary to a single Class II HLA locus.

Brief Description of the Drawings

In the drawings:

Fig 1 is an autoradiograph described in Example 4;

Fig 2 is an autoradiograph described in Example 6;

Fig 3 is a restriction map of the HLA—DRα locus showing the location of polymorphic restriction sites for the enzymes BglII and EcoRV detected with the probe of Example 1;

Fig 4 is an autoradiograph described in Example 7;

Figs 5, 6, and 7 are the autoradiographs described in Example 8; and

Fig 8 and 9 are the autoradiographs described in Example 9.

Modes for Carrying Out the Invention

The initial step in typing an individual's HLA by the invention method is to obtain a sample of the individual's genomic HLA DNA. As used herein, the term "individual" is intended to include beings that are in a fetal stage. All nucleated cells contain HLA DNA and, therefore, are potential sources for the required DNA. For convenience peripheral blood cells will typically be used rather than tissue samples. As little as 10 to 100 cc of peripheral blood provide sufficient HLA DNA for typing. In the case of fetal HLA typing, placental cells or amniotic fluid may be used. The DNA is isolated from nucleated cells under conditions that preclude DNA degradation. Such isolation involves digesting the cells with a protease that does not attack DNA at a temperature and pH that reduces the likelihood of DNase activity followed by extraction of the digest with a DNA solvent. DNA isolation from nucleated cells is described by Kan, et al, *N Eng J Med* (1977) 297:1080—1084 and *Nature* (1974) 251:392—393, and Kan and Dozy, supra. The extracted DNA may be purified by dialysis, chromatography, or other known methods for purifying polynucleotides.

In the second step of the method the isolated DNA is restricted with a restriction endonuclease that cleaves or cuts DNA hydrolytically at a specific nucleotide sequence. Sequences so recognized by the enzymes are called restriction sites. Restriction endonucleases that recognize and cleave at specific sites are sometimes referred to as class II restriction enzymes (class I enzymes cleave randomly rather than at specific sites). Enzymes that produce blunt end DNA fragments (hydrolysis of the phosphodiester bonds on both DNA strands occur at the same site) as well as enzymes that produce sticky ended fragments (the hydrolysis sites on the strands are separated by a few nucleotides from each other) may be used. In any event, it is essential that the restriction endonuclease be one that produces a polymorphic digestion pattern associated with the Class II HLA locus or loci under investigation. Determinations of which enzymes produce RFLPs at which loci may be made experimentally using various enzymes in conjunction with various specific HLA cDNA probes in the invention method. Table 1 lists the RFLPs that have been identified to date in this manner.

TABLE 1

| HLA cDNA probe | Enzymes revealing RFLP |
| --- | --- |
| pHLA-Dp34 (DRα) | *Bg*/II, *Eco*RV |
| p29G8 (DRα-related) | *Bg*/II |
| pHLA-B7 | *Eco*RI, *Pvu*II, *Kpn*I, *Xba*I, *Hind*III, *Bam*HI |
| pHLA-DRβ-4 (DRβ-related) | *Eco*RI, *Bg*/II |
| pHLA-DRβ-8 | *Eco*RI, *Bg*/II |

The digestion of the DNA with the endonuclease may be carried out in an aqueous medium under conditions favoring endonuclease activity. Typically the medium is buffered to a pH of 6.5 to 8.0. Mild temperatures, 20°C to 45°C, preferably physiological temperatures, are employed. Restriction endonucleases normally require magnesium ions and, in some instances cofactors (ATP and S-adenosyl methionine) or other agents for their activity. Therefore, a source of such ions, for instance inorganic magnesium salts, and other agents, when required, will be present in the medium. The amount of DNA in the digestion mixture will typically be in the range of 1% to 20% by weight. In most instances 5 to 20 µg of total cell DNA digested to completion provides an adequate sample for typing. Excess endonuclease, usually one to five units/µg DNA, will be used. If desired the restriction digest may be worked up by precipitation and resuspension as described by Kan and Dozy, supra, prior to being subjected to genomic blotting.

The third step of the process is analyzing the restriction digest by genomic blotting for the presence of one or more HLA gene sequences. In the case of typing for a particular HLA gene the analysis is directed to detecting a DNA sequence that uniquely characterizes that gene. However, when the invention is used for paternity testing or transplant or transfusion compatibility the analysis does not necessarily involve identifying a specific locus or loci but may be done by comparing single or multilocus patterns of one individual with that of another individual using the same restriction endonuclease and an equivalent probe to determine similarities and differences between the patterns. In this regard a single locus probe will identify RFLPs associated with a single HLA locus whereas a multilocus probe will identify RFLPs associated with two or more HLA loci. Three basic steps are involved in the analysis: (1) separating the fragments by size; (2) annealing the fragments with a labeled cDNA probe that is complementary to the desired HLA DNA sequence(s); and (3) detecting the presence of labeled hybrids. The genomic blotting embodiment uses the indicated sequence. The analysis method known as "Southern blotting" that is described by Southern, E.M. (1975) *J Mol Biol* 98:503—517 is currently a preferred analysis method. In Southern blotting the digestion products are electrophoresed, transferred and affixed to a support which binds nucleic acid, and hybridized with an appropriate labeled cDNA probe. Labeled hybrids are detected by autoradiography.

Electrophoresis is the separation of the digestion products contained in a supporting medium by size under the influence of an applied electric field. Gel sheets or slabs, eg agarose or agarose-acrylamide, are typically used as the supporting medium in Southern blotting. The electrophoresis conditions are such as to effect the desired degree of resolution of the fragments. A degree of resolution that separates fragments that differ in size from one another by as little as 100 base pairs will usually be sufficient. Sizes markers are run on the same gel to permit estimation of the size of the restriction fragments. In carrying out the electrophoresis, the digestion products are loaded onto one end of the gel slab (commonly called the "origin") and the fragments separate by electrically facilitated transport through the gel, with the shortest fragment electrophoresing from the origin towards the other (anode) end of the slab fastest.

After electrophoresis the gel is readied for annealing by placing it in a DNA denaturing solution, conveniently a mild base, generally about 0.2 to 1 M hydroxide, preferably 0.5 M NaOH, to dissociate the DNA strands. After denaturation, the gel is placed in a neutralizing solution and neutralized to a mildly acid pH. The DNA is then transferred to the substrate, which is typically made from materials such as nitrocellulose paper or diazobenzyloxymethyl paper, by contacting the gel with the paper in the presence of reagents, eg buffer, and under conditions, eg light weight and 0°C to 25°C, that promote transfer and covalent or noncovalent binding of the DNA (particularly guanosine and uridine bases) to the sheets. Such reagents and conditions are described by Southern, E.M. supra, Wahl, et al, *PNAS* (1979) 76:3683—3687, Kan and Dozy, supra, and US 4,302,204. After the transfer is complete the paper is separated from the gel and is dried. Hybridization (annealing) of the resolved single strand DNA on the paper to an HLA cDNA probe is effected by incubating the paper with the probe under hybridizing conditions. The hybridization

5

will typically be conducted in an aqueous buffer solution containing a polar solvent such as formamide. Other additives that enhance the hybridization such as sodium chloride, sodium citrate, serum albumin and sonicated denatured DNA such as denatured salmon sperm DNA may be included in the hybridization medium. See Southern, supra, Kan and Dozy, supra and US Pat No 4,302,204, col 5, line 8 et seq.

Complementary DNA probes that are specific to one (locus specific) or more (multilocus) particular HLA DNA sequences involved in the polymorphism are essential components of the hybridization step of the typing method. Locus specific cDNA probes may be made by identifying desired HLA cDNA clones in cDNA libraries constructed from membrane-bound mRNA using synthetic oligonucleotide probes that hybridize to specific HLA cDNA clones. The clones are made detectable by labeling them with a detectable atom, radical or ligand using known labeling techniques. Radiolabels, preferably $^{32}$P, will typically be used. The identified clones may be labeled with $^{32}$P by nick translation with an $\alpha$-$^{32}$P-dNTP (Rigby, et al, *J Mol Biol* (1977) 113:237) or other available procedures to make the locus specific probes for use in the invention methods.

The base sequences for the synthetic oligonucleotide probes used to screen the cDNA libraries are determined from HLA antigen amino acid sequences using the genetic code. The amino acid sequences may be determined experimentally or from published data eg, Ploegh, et al, supra and Sood, et al, supra. Amino acids with minimal codon degeneracy are used whenever possible. If the amino acid sequence suggests more than one possible oligonucleotide sequence, all possible oligonucleotide sequences that code for the amino acid sequence are made and tested to determine which results in the best probe. Oligonucleotides having the desired base sequences may be prepared using the known phosphate diester, phosphate triester, or phosphite triester techniques. The phosphate triester method described by Good, et al, *Nucl Acid Res* (1977) 4:2157, is preferred. Blocked nucleotide reagents used in this method are available commercially. The specificity of a synthetic oligonucleotide may be determined by primer extension analysis or by using it as a hybridization probe in "Northern" blot analysis (a technique analogous to the Southern blot method for analyzing mRNA instead of DNA that was developed by Alwine, et al, *PNAS* (1977) 74:5350) of poly(A[+]) mRNA from a B cell line. Potential locus specific probes may also be identified by hybridizing cDNA library clones with multilocus probes and determining the specificity of the clones that anneal with the multilocus probes.

As more information about the DNA sequences of HLA genes becomes available it will be possible to discern whether there are any sequences that are unique to a particular locus, or perhaps a particular allele. Such information will permit the synthesis of locus specific probes that hybridize only to the unique portion of the gene. In this regard preliminary data have been developed indicating that in the case of the HLA—A and HLA—B loci the distinguishing portions of the genes may lie in the 3' untranslated regions.

The final step in the method is identifying labeled hybrids on the paper (or gel in the solution hybridization embodiment). Autoradiography is currently used to detect radiolabel-containing hybrids. It involves laying the paper on a piece of radiation-sensitive film (X-ray film). The disintegration of the label results in the deposition of a silver grain in the film. The film is developed and the pattern of labeled fragments is identified. The specificity of the probe and the particular restriction endonuclease used will determine the number of fragments that appear in the pattern. Locus specific probes will typically give patterns with fewer bands than the patterns produced using multilocus probes.

As indicated previously these autoradiographs are used to determine HLA type or, in the case of paternity testing, transplant or transfusion compatibility, and disease association to determine similarities in the autoradiograph patterns of different individuals or similarities between an individual's pattern and a standard pattern, as the case may be. In this regard it will be appreciated that paternity testing and transplant or transfusion compatibility may also be carried out by HLA typing the individuals by the invention method and comparing their HLA types. In HLA typing the fragment(s) appearing on the test autoradiograph is/are compared to the fragment(s) that characterize a particular HLA type to determine correspondence therebetween and thus whether the test subject is that HLA type. This may be done by matching the test autoradiograph with a standard autoradiograph or simply matching the size distribution of the fragment(s) appearing on the test autoradiograph with the size distribution of fragments(s) for the standard. By evaluating the HLA DNA RPLF patterns for individuals of known (by conventional HLA typing) HLA type it is possible to assign specific restriction fragments to a given HLA locus. It is also expected that practice of the invention method will identify hitherto unidentified HLA genes, particularly in Class II. In this manner correlations between restriction fragment patterns and HLA type may and will be made. Such correlations may be used in deciphering test autoradiographs. The method also provides a technique for defining subdivision of serologically defined HLA types. The use of HLA types in paternity tests, transplantation or transfusion testing and in disease diagnosis and prognosis is described in *Basic & Clinical Immunology,* 3rd Ed (1980) Lange Medical Publications, pp 187—190.

HLA cDNAs identified as potential probes may also be useful in making recombinant clones expressing human HLA antigens. In this connection, cDNA that encodes a given HLA antigen is inserted into an appropriate cloning vehicle (vector) and hosts are transformed with the vehicles. Transformants are isolated and those that produce the desired antigen are cloned. The antigen may be harvested from the clones by conventional methods. Such antigens may be useful for diagnostic purposes, for making anti-HLA antibodies, or for therapy to induce tolerance to HLA antigens.

The following examples further illustrate the various aspects of the invention. These examples are not intended to limit the invention in any manner.

Example 1
Preparation of Hybridization Probe for HLA—Dp34 (HLA—DRα)

Four 11-mer oligonucleotides were prepared based on the known $NH_2$-terminal amino acid sequence (*Glu, Phe, Tyr, Leu*) of positions 11—14 of HLA-Dp34 antigen. The base sequences for the four oligonucleotides were as follows: (1) *AGGTAAAATTC,* (2) *AGGTAGAATTC,* (3) *AGGTAAAACTC,* and (4) *AGGTAGAACTC.* These sequences are all complementary to the codons for the indicated peptide sequence and were chosen to minimize degeneracy. The ambiguities are located at sequence positions 2, 3, 6, and 9. A *G* at positions 2 and 3 was chosen to minimize the destabilization effect of potential mismatched bases (*G* is capable of forming a wobble pair with *U*).

Since the four oligonucleotides were complementary to codons for amino acids 11—14, oligonucleotide primed cDNA synthesis on HLA-Dp34 mRNA was expected to generate a product of about 150—200 nucleotides. This estimate was based on a leader sequence of ~75 nucleotides and assumes a 5′ untranslated region of 75—125 nucleotides.

The specificities of the four 11-mers were compared by using them individually as primers in cDNA synthesis reactions using membrane-bound B cell mRNA, free B cell mRNA, and T cell mRNA as template. Only the *AGGTAGAACTC* oligonucleotide primed a cDNA band of ~175 nucleotides which was enriched in reactions on B cell membrane-bound mRNA template. The specificity of this 11-mer oligonucleotide was confirmed by extending the primer in a cDNA synthesis reaction in the presence of a single dideoxy triphosphate and three deoxy triphosphates, an approach which has proved successful in the isolation of the HLA-B7 cDNA clone (Sood, et al, supra). In the presence of dideoxy dATP, a minor cDNA band corresponding to a predicted 18-nucleotide primer extension product was observed. The additional seven nucleotides were determined by the wandering spot sequencing technique to be *GGCCTGA.* The following two additional nucleotides, *AT,* were inferred from the Ile codon, giving a nine nucleotide sequence that corresponded to the HLA-Dp34 antigen amino acid at positions 8, 9 and 10.

A 20-nucleotide fragment having the above determined sequence *(AGGTAGAACTCGGCCTGAAT)* was then synthesized by the triester method. The specificity of the 20-mer as a primer was examined in a cDNA synthesis reaction on a poly($A^+$) mRNA from a B cell line. A major cDNA band, 175 nucleotides long, was synthesized; the nucleotide sequence of the eluted band, corresponded to the expected sequence for HLA-Dp34.

The specificity of the 20-nucleotide fragment as a hybridization probe was analyzed on a Northern blot of poly($A^+$) mRNA. A unique band, at 1200—1300 nucleotides resulted from probing B cell mRNA but not T cell mRNA with the $^{32}$P-labeled 20-mer nucleotide probe. Membrane-bound mRNA was enriched for the mRNA which hybridized to the 20-nucleotide probe.

An HLA-Dp34 cDNA clone was identified in a cDNA library with the above described 20-mer probe as follows. Membrane-bound RNA and free RNA was prepared, using phenol-chloroform extraction in the presence of Vanadyl complexes, from the human lymphoblastoid B cell line, CA. Poly($A^+$) mRNA, isolated by affinity chromatography with Poly U-Sepharose, was translated in an *in vitro* rabbit reticulocyte system. The partition of specific mRNA's into the membrane-bound and free fractions was monitored by 2D gel analysis of the $^{35}$S-labeled products of *in vitro* translation. A double-stranded cDNA library was prepared from the membrane-bound mRNA using reverse transcriptase, DNA Polymerase I, and SI nuclease. Following tailing with dCTP using terminal transferase, the cDNA was inserted and ligated to preparations of the plasmid pBR322 which had been digested with *Pst* and tailed with dGTP.

Initial screening of the library was carried out as follows. Duplicate sets (~4,000 clones/set) of Grunstein-Hogness colony filters were prepared. One set was probed with $^{32}$P cDNA made from size fractionated mRNA from the B cell line, CA. Sucrose gradient fractions were translated in an *in vitro* rabbit reticulocyte system and the $^{35}$S-labeled products analyzed by 2D gel electrophoresis to determine the appropriate fraction. The other set of filters was probed with $^{32}$P cDNA made from mRNA from the T cell line, Molt-4. A subset of about 150 clones, derived from membrane-bound, B cell specific, 12—14s mRNA, was defined by this initial screening.

Plasmid DNA was prepared from 25 pools, each consisting of 5 candidate cDNA clones and analyzed by dot hybridization with the $^{32}$P-labeled 20-nucleotide probe. Pool 14 plasmid DNA hybridized specifically with the .probe. Subsequently, the individual members of the pool were tested; cDNA sequences complementary to the hybridization probe were restricted to the clone identified as 18C7.

In Northern blots, the $^{32}$P-labeled 18C7 nick translated probe hybridizes to a B cell mRNA of the same length (about 1200 to about 1300 nucleotides) as the band complementary to the 20-nucleotide probe. In genomic blots with DNA from a hamster-human hybrid containing the human chromosomes 6 and 3, the 18C7 probe hybridizes to a unique restriction fragment absent in the hamster parent, mapping the 18C7 DNA sequences to chromosome 6.

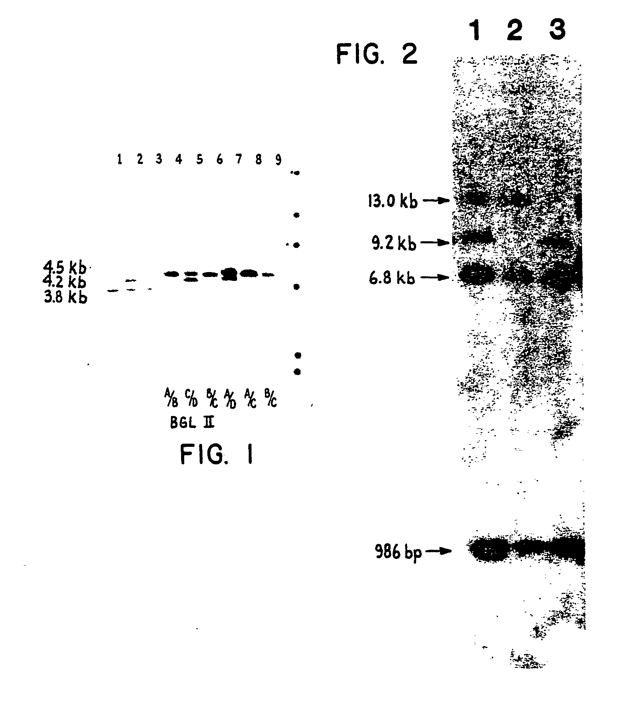

A more precise mapping was possible using the cell line 6.3.6 which has a small deletion at a defined site on the short arm of one homologue of the chromosome 6 pair. This deletion variant fails to express the HLA—A, B, C and HLA-DR specificities associated with one chromosome 6 haplotype. In genomic blots (Fig 1 and Example 4 below), 18C7 hybridizes to two restriction fragments from the parent cell line, presumably from the two chromosome 6's. Only one fragment is observed in DNA from the deletion variant; the other fragment is presumably derived from the chromosome which has been deleted. This result maps DNA sequences complementary to the 18C7 clone to the chromosomal site defined by the 6.3.6 deletion.

The human HLA-D locus is homologous to the mouse I region. In genomic blots with DNA from mouse congenic lines, inbred lines which differ only at the I region, the 18C7 probe hybridized to a restriction fragment that was different with each congenic line. This result maps DNA sequences complementary to the 18C7 clone to the mouse I region and therefore to the human HLA-D locus.

The 18C7 clone was confirmed as being HLA-Dp34 (HLA-DRα) by analyzing its DNA sequence by the Maxam-Gilbert technique *(Methods in Enzymology* (1980) 65:499—560) using the endonucleases *Pst*I, *Hin*fI, *Taq*I, *Sau*3A, *Ava*II, and *Bgl*I. The sequence for the coding strand of the HLA-Dp34 clone is given below (N = unidentified nucleotide).

```
ATCATAGCTG   TGCTGATGAG   CGCTCAGGAA   TCATGGGCTA   TCAAAGAAGA
ACATGTGATC   ATCCAGGCCG   AGTTCTATCT   GAATCCTGAC   CAATCAGGCG
AGTTTATGTT   TGACTTTGAT   GGTGATGAGA   TTTTCCATGT   GGATATGGCA
AAGAAGGAGA   CGGTCTGGCG   GCTTGAAGAA   TTTGGACGAT   TTGCCAGCTT
TGAGGCTCAA   GGTGCATTGG   CCAACATAGC   TGTGGACAAA   GCCAACCTGG
AAATCATGAC   AAAGCGCTCC   AACTATACTC   CGATCACCAA   TGTACCTCCA
GAGGTAACTG   TGCTCACGAA   CAGCCCTGTG   GAACTGAGAG   AGCCCAACGT
CCTCATCTGT   TTCATCGACA   AGTTCACCCC   ACCAGTGGTC   AATGTCACGT
GGCTTCGAAA   TGGAAAACCT   GTCACCACAG   GAGTGTCAGA   GACAGTCTTC
CTGCCCAGGG   AAGACCACCT   TTTCCGCAAG   TTCCACTATC   TCCCCTTCCT
GCCCTCAACT   GAGGACGTTT   ACGACTGCAG   GGTGGAGCAC   TGNGGCTTGG
ATGAGCCTCT   TCTCAAGCAC   TGGGAGTTTG   ATGCTCCAAG   CCCTCTCCCA
GAGACTACAG   AGAACGTGGT   GTGTGCCCTG   GGCCTGACTG   TGGGTCTGGT
GGGCATCATT   ATTGGGACCA   TCTTCATCAT   CAAGGGAGTG   CGCAAAAGCA
ATGCAGCAGA   ACGCAGGGGG   CCTCTGTAAG   GCACATGGAG   GTGATGATGT
TTCTTAGAGA   GAAGATCACT   GAAGAAACTT   CTGCTTTAAT   GACTTTACAA
AGCTGGCAAT   ATTACAATCC   TTGACCTCAG   TGAAAGCAGT   CATCTTCAGC
GTTTTCCAGC   CCTATAGCCA   CCCCAAGTGT   GGTTATGCCT   CCTCGATTGC
TCCGTACTCT   AACATCTAGC   TGGCTTCCCT   GTCTATTGCC   TTTTCCTGTA
TCTATTTTCC   TCTATTTCCT   ATCATTTTAT   TATCACCATG   CAATGCCTCT
GGAATAAAAC   ATACAGGAGT   CTGTCTCTGC   TATGGAATGC   CCCATGGGGC
TCTCTTGTGT   ACTTATTGTT   TAAGGTTTCC   TCAAACTGTG   ATTTTTCTG
```

A $^{32}$P-labeled HLA-Do34 probe was made from the clone by nick translation.

Example 2

Preparation of Hybridization Probe for Ia Like Class II HLA p29G8

The HLA-DRα related or human Ia like clone, p29G8, was identified by screening the cDNA library of Example 1 with the nick-translated HLA-Dp34 (DRα) probe under hybridization conditions of reduced stringency to allow detection of related but distinct DNA sequences. The hybridization conditions were as follows.

Hybridize in 50% formamide, 5 × SSPE (1 × SSPE = 0.18 M NaCl, 10 mM $NaH_2PO_4$, 1 mM $Na_2EDTA$, pH 7.0), 0.1% sodium dodecyl sulfate (SDS), 5 × Denhardt's (5 × Denhardt's = 0.1% w/v each bovine serum albumin, Ficoll, polyvinyl pyrollidone), 200 µg/ml sheared denatured salmon sperm DNA, at 37°C for 24 h with 1 × $10^6$ cpm [32]p-labeled HLA-Dp34 probe (2 × $10^8$ cpm/µg, labeled by nick translation). Wash filters 3 × 15 min at room temperature in 5 × SSPE, 0.1% SDS.

Fifteen HLA-DRα related cDNA clones were identified. Among them was a clone designated p29G8.

Under conditions of high stringency (wash at 0.1 × SSPE, 65°C), p298G hybridizes strongly only to itself. The p29G8 clone (minus strand) was partially sequenced using the Maxam-Gilbert procedure. Four fragments, designated A1, A2, B1, and B2, were obtained by digesting the p29G8 clone. The clone was first digested with PstI to yield a ⁓400 bp fragment and a ⁓600 bp fragment. The larger fragment was cut with Sau3A to give fragment A1 or with MspI to give fragment A2. The smaller fragment was cut with HaeIII to give fragment B1 or with Sau3A to give fragment B2. The sequence of these fragments are reported below (N = unidentified nucleotide).

```
Fragment A1

TNTGAACNCCAGCTGCCCTACAAACTCCATCTCAGCTTTTCTTCTCACTTCATG

TNAAAACTACTCCAGTGGCTGACTNAATTGCTGACCCTTCAAGCTCTGTCCTTA

TCCATTACCTCAAAGCAGTCATTCCTTAGTNAAGTTTCCAAC

Fragment A2

CACGGGAGNCCCAAGAGCCAACCAGACGCCTGAGACAACGGAGACTGTGCTCTG

TGCCCTGGGCCTGGTGCTGGGCCTAGTCGGCATCATCGTGGGCACCGTCCTNAT

CATAAAGTCTCTGCGTTCTGGCCATGACCCCC

Fragment B1

CACATTGACGAGTTCTTCCCACCAGTCCTCAANGTCACGTGGGCCGCGCAACGG

GGAGCTGGTCACTGAGGG

Fragment B2

AAGGAGACCGTCTGGCATCTGGAGGAGTTTGGCCAAGCCTTTTCCCTTTGAGGC

TCAGGGCGGGCTGGCTAACATTGCTATATTGAACAACAACTTGAAACCTTGA
```

The partial sequence is distinct from the sequence for HLA-Dp34 (DRα) and differs from the recently published nucleotide sequence for the HLA-DCI clone (Auffrey, et al, (1982) *PNAS* Vol 79:6637—6341) both in sequence and length of the 3' untranslated region. In genomic Southern blots, the p29G8 probe hybridizes to genomic restriction fragments distinct from those which hybridize to the HLA-Dp34 (DRα) probe in DNA from an HLA hemizygous cell line (6.3.6). This observation indicates that p298G represents a different (new) locus and not simply another allele. The genomic blot pattern with DNA from the cell line T5—1 and its HLA hemizygous derivative 6.3.6 indicates that the p29G8 locus maps within the HLA region.

Example 3
Characterization of Other Class II HLA cDNA Clones

DRα

As indicated in Example 2, fourteen other DRα-related cDNA clones were identified. Four of these clones are very strongly homologous to the HLA-Dp34 (DRα) clone and are probably identical or allelic copies. Thus, ten clones represent candidates for new HLA-DRα related loci. These ten clones are listed below.

| | |
|---|---|
| p14A9 | p11G7 |
| p17D8 | p36A1 |
| p22G12 | p51H2 |
| p40G4 | p25A6 |
| p6B3 | p36F7 |

Clone p14A9 hybridizes to genomic restriction fragments distinct from those that hybridize to the HLA-Dp34 (DRα) and p29G8 probes. Thus, p14A9 represents a third DRα related locus. By comparing the pattern of cross-hybridization of these HLA-DRα related clones, it was estimated that these 10 HLA-DRα related clones represent a minimum of two and a maximum of 10 different HLA-DRα related loci.

DRβ

An 18-mer having the base sequence *CCCTGTCTCGCGCACGCA* was prepared for use in screening a cDNA library of HLA-DRβ clones. The sequence of the 18-mer was based on the published amino acid sequence for the conserved amino acids 20—25 of the HLA-DRβ chain. The specific 18-nucleotide sequence was chosen from the published sequence of an HLA-DRβ cDNA clone from the Raji cell line (Wiman et al (1982) *PNAS* 79:1703—1707). The specificity of the kinased 18-mer probe was tested by hybridization to an RNA blot. The probe hybridized to an RNA species about 1100—1300 nucleotides long present in B cell RNA and absent from T cell RNA, as expected for the HLA-DRβ mRNA. Hybridization conditions were for 36 h at 37°C in 4 × SSPE with 5 × Denhardt's solution, 0.2 mM ethylene diaminetetraacetic acid (EDTA), and 0.1% SDS. Filters were washed with 2 × SSPE, 0.1% SDS at room temperature.

The 18-mer probe was hybridized to a cDNA bank derived from mRNA from the β lymphoblastoid cell line, LG2. The cDNA bank was constructed by inserting the duplux cDNA ligated to EcoRI linkers into the EcoRI site of the λgt 10 vector. cDNA inserts from eight 18-mer reactive λ cDNA clones were isolated and subcloned into the EcoRI site of the plasmid vector, pBR328 (Soberon, X., et al, Gene (1980) 9:287—305). The clones designated DRβ-4 and DRβ-8 hybridize to sequences in the HLA region using the genomic blotting technique with the 6.3.6. HLA hemizygous deletion variant described in Example 1. The products of in vitro translation of mRNA which hybridize to DRβ chains have the electrophoretic mobility expected of HLA-DRβ chains. In oocytes, the translation products of specifically hybridizing mRNAs associate with the translation products of HLA-DRα and HLA-DRγ mRNAs. Thus, by a variety of criteria, these clones encode HLA-DRβ or DRβ like proteins. The genomic blot patterns obtained with the HLA-DRβ-4 and HLA-DRβ-8 probes are different indicating these clones represent different loci.

## Example 4
### Determination of HLA-DRα Restriction Fragment Length Polymorphism Using BglII and HLA-Dp34 Hybridization Probe

Digestion of DNA with BglII

Samples of DNA were obtained from nucleated cells of five unrelated individuals and four children of two of the individuals. Five to ten μg of each DNA sample were digested for 1—2 hr at 37°C with 4(15') units of BglII per μg of DNA. The buffer was 60 mM NaCl, 6 mM Tris HCl (pH 7.5), 6 mM $MgCl_2$, 6 mM 2-mercapto-ethanol.

Genomic Blotting of Restriction Digests with HLA-Dp34 Probe

Between five and ten μg of each restriction enzyme digested DNA was fractionated according to size by electrophoresis in a 0.6% agarose gel for approximately 500 V-hr. Electrophoresis buffer was 40.0 mM Tris, 2.5 mM EDTA, 25.0 mM acetate, pH 8.2. After electrophoresis the DNA was stained with 0.5 μg/ml ethidium bromide and the gel was photographed. The DNA in the gel was depurinated with a 15' wash in 75 mM HCl at room temperature. DNA was denatured with 2 successive 15' washes in 0.5 M NaOH + 1.5 M NaCl at room temperature. DNA was neutralized with 2 successive 15' washes in 1.5 M Tris-Cl pH 7.4 + 3.0 M NaCl at room temperature. DNA was transferred from the gel to nitrocelluluse (0.45 micron pore size) by blotting with 20 × SSPE (20 × SSPE = 3.6 M NaCl, 200.0 mM phosphate, 20.0 mM $Na_2EDTA$, pH 7.0) for 3 hr at room temperature. DNA was bound to the nitrocellulose by baking at 80°C for 2 hr in a vacuum oven.

The nitrocellulose filter was placed in a heat sealable plastic bag and was prehybridized for 8 hr at 42°C in a solution composed of 50% formamide, 5 × SSPE, 200 μg/ml sheared denatured salmon sperm DNA, 0.1% SDS, and 2 × Denhardt's solution. After 8 hr, the prehybridization solution was removed from the bag and replaced with a solution composed of 50% formamide, 5 × SSPE, 100 μg/ml sheared denatured salmon sperm DNA, 0.1% SDS, 2 × Denhardt's solution, 10% sodium dextran sulphate, and $1$—$5 × 10^6$ cpm denatured $^{32}P$ labelled HLA-Dp34 probe. The probe was labeled using the nick translation reaction of Rigby, et al, supra, to specific activities of $5 × 10^8$ $-1 × 10^9$ cpm/μg. The bag was resealed and the nitrocellulose filter was hybridized with the probe for 18—24 hr at 42°C.

The nitrocellulose filter was removed from the bag and washed in 4 successive changes (15' each) of 2 × SSPE, 0.17% SDS at room temperature. The filter was then washed in 4 successive changes (15' each) of 0.1 × SSPE, 0.1% SDS at 50°C.

The filter was air dried, covered with Saran Wrap and autoradiographed with Kodak XAR—5 film with an intensifying screen for 18—72 hr at −80°C. Fig 1 is a copy of the resulting autoradiograph.

Discussion of Autoradiograph

The autoradiograph shows the BglII produced three different restriction fragments in the tests: 3.8, 4.2, and 4.5 kb in length. This clearly evidences that the HLA-Dp34 locus is polymorphic and that there are at least three alleles of this gene.

Lane-1 of the autoradiograph is a blot of the cell line CA. Lanes 3 and 2 of the autoradiograph are blots of the cell line 6.3.6 and its parent T5—1, respectively. These are the blots discussed in Example 1 that map the HLA-Dp34 gene to chromosome 6 at the site defined by the 6.3.6 deletion.

Lanes 4 and 5 are blots of the mother and father of a family and lanes 6—9 are blots of the children of those parents. Lane 4 is the father's blot and his haplotype is designated A/B. Both chromosome A and chromosome B have the same restriction fragment (4.2 kb). Lane 5 is the mother's blot and her haplotype is designated C/D. Each chromosome C and D has a different restriction fragment (4.2 kb and 4.5 kb, respectively). In the offspring of these parents the maternal 4.2 kb genomic fragment segregates with the serologically defined D haplotype.

## Example 5
### HLA-Dp34 Typing Based on RFLB Using HLA-Dp34 Hybridization Probe and the Restriction Endonuclease *Bg*lI

A sample of peripheral blood is obtained from an individual and the HLA DNA is extracted therefrom using the methods described in Example 4. The DNA is digested using *Bg*lI and a genomic blot of the digest is made using the HLA-Dp34 as described in Example 4. The restriction fragment pattern of the resulting autoradiograph is compared to the restriction fragment patterns described in Example 4 (Fig. 1) for HLA-Dp34 to determine the individual's HLA-Dp34 type.

## Example 6
### Restriction Fragment Length Polymorphisms Detected with *Eco*RV and HLA-Dp34 (DRα) Probe

Ten µg samples of DNA were obtained from three individuals and digested for 1—4 hr at 37°C with 80 units of *Eco*RV. The buffer was 0.15 M NaCl, 6 mM Tris HCl (pH 7.9), 6 mM $MgCl_2$, 6 mM 2-mercaptoethanol. Genomic blotting and hybridization of restriction digested DNAs were carried out as in Example 4. An autoradiograph was prepared of the genomic blotting patterns as in Example 4. Fig 2 is a copy of the resulting autoradiograph.

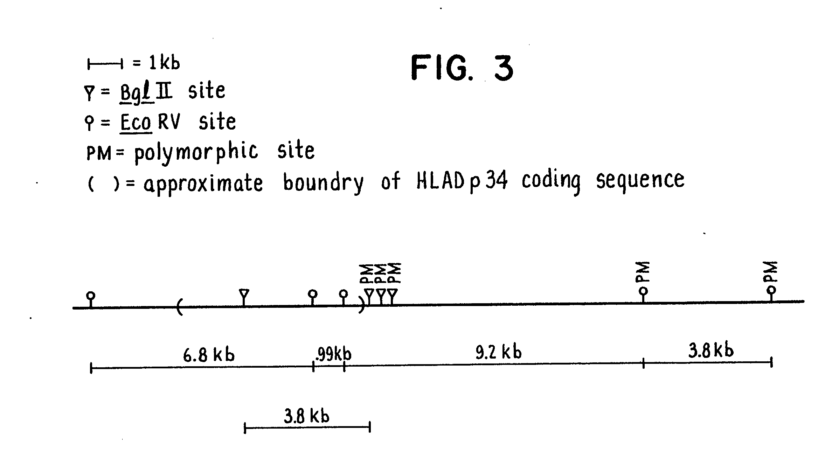

As seen in Fig 2, all three individuals possess a 986 bp and a 6.8 kb *Eco*RV fragment (nonpolymorphic fragments). In addition, every individual possesses either a 9.2 kb or a 13.0 kb *Eco*RV fragment, or both (polymorphic fragments). Fig. 3 is a map diagramming the location of the polymorphic restriction sites for *Bg*lI (Example 4) and *Eco*RV detected with the HLA-Dp34 probe.

## Example 7
### Determination of Restriction Fragment Length Polymorphism in Class II Locus Using *Bg*lI and Hybridization Probe p29G8

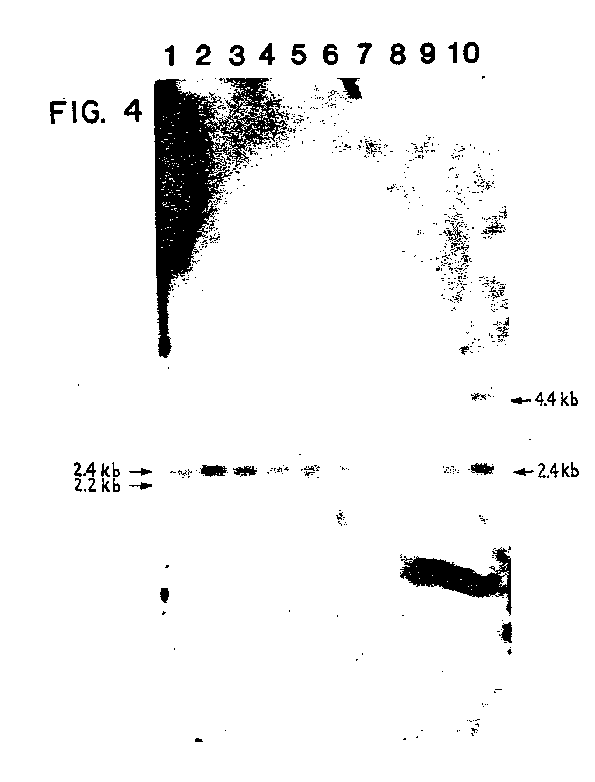

Ten µg samples of DNA from five individuals of one family and five individuals of a second family were digested with *Bg*lI using the procedure of Example 4. The digests were subjected to electrophoresis, genomic blotting and hybridization by the procedure of Example 4 except that the nick translated p29G8 probe of Example 2 was used instead of the HLA-Dp34 probe. Fig 4 is an autoradiograph of the resulting genomic blotting patterns. Lanes 1—5 are the patterns for the five individuals of the first family and Lanes 6—10 are the patterns of the five individuals of the second family. As seen in Fig 4 three types ("alleles") were observed in the samples characterized by 2.2 kb, 2.4 kb, and 4.4 kb fragments. In the HLA typed families of this example the polymorphic *Bg*lI fragments segregate with serologically defined parental haplotypes.

## Example 8
### Use of HLA—B7 Hybridization Probe to Evaluate HLA Restriction Fragment Length Polymorphisms in Human Pedigrees

An HLA-B7 cDNA clone was obtained from Sherman M. Weissman, Dept of Human Genetics, Yale University School of Medicine. This clone is described in Sood, et al, supra.

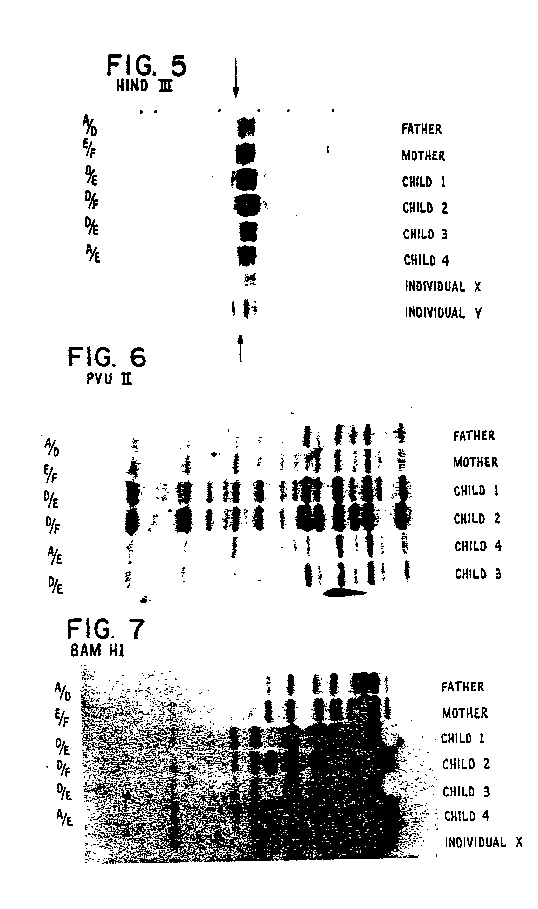

DNA samples from the father, mother, and four children of an HLA typed family and in two instances DNA from one or two individuals (designated X and Y) unrelated to that family were digested according to Example 4 with either *Hind*lII, *PVU*lI, or *Bam*HI. Genomic blots of the digests were made as in Example 4 using the [32]p nick translated HLA-B7 clone as a hybridization probe. Figs 5, 6 and 7 are copies of the resulting autoradiographs. The father's haplotype is designated A/D and the mother's haplotype is designated E/F. These autoradiographs indicate that the probe is a multilocus probe that hybridizes to more than one HLA locus. Nontheless, several polymorphic bands, segregating in the pedigree, are present for each enzyme used. Moreover, the bands segregate with the serologically defined HLA loci so that given fragments may be assigned to an individual chromosome.

Exclusionary paternity determinations may be made using autoradiographs such as Figs 5, 6 and 7. An exclusionary pattern would involve a restriction fragment pattern which could not be inherited from the mother and the alleged father. Such a pattern would be one in which the child has a fragment that neither the mother nor the alleged father has. Positive paternity determinations using RFLPs will depend upon the frequency of the RFLPs in the general population. In such determinations one calculates the probability that the putative father contributed the RFLP that is observed and compares it with the probability that any random male would contribute the RFLP to the child.

## Example 9
### Use of *Bg*lI and HLA-Dp34 (DRα) Probe to Evaluate Linkage and Association Between HLA-DRα and Insulin Dependent Diabetes Mellitus (IDDM) in Six Families

Ten µg samples of DNA from the individuals of six different families having histories of IDDM were digested with *Bg*lI, blotted, and hybridized with the HLA-Dp34 probe by the procedure of Example 4. Autoradiographs of the resulting genomic blotting patterns were made.

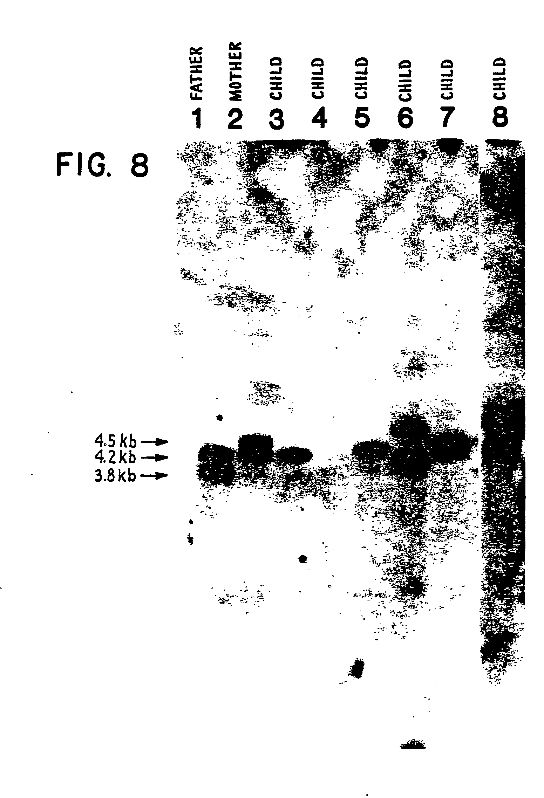

Fig 8 is an autoradiograph of eight individuals of one of the families. The father's pattern appears in Lane 1, the mother's in Lane 2 and six children in Lanes 3—8. The father has the 4.2 kb fragment (derived from chromosome 6 haplotype A) and the 3.8 kb fragment from haplotype B. The mother has the 4.2 kb fragment (haplotype C) and the 4.5 kb fragment (haplotype D). The three affected (IDDM) children have either two copies of the 4.2 kb fragment (haplotype A/C, Lanes 3 and 5) or one copy of the 4.2 kb fragments (haplotype A) and one copy of the 4.5 kb fragment (haplotype D) in Lane 4. One unaffected child (Lane 7) has two copies of the 4.2 kb fragment (haplotype A/C). The two unaffected children (Lanes 6 and 8) both have the 3.8 kb fragment (haplotype B) and the 4.5 kb fragment (haplotype D). Thus, the 4.2 kb fragment is linked in this family to a disease susceptibility gene for IDDM. Three of the four children with the 4.2 kb fragment exhibit IDDM, indicating an incomplete penetrance (genetic predisposition) of the disease allele.

In four of the other five families tested the segregation pattern was consistent with linkage of the 4.2 kb *Bg/*II fragment to the IDDM disease susceptibility allele.

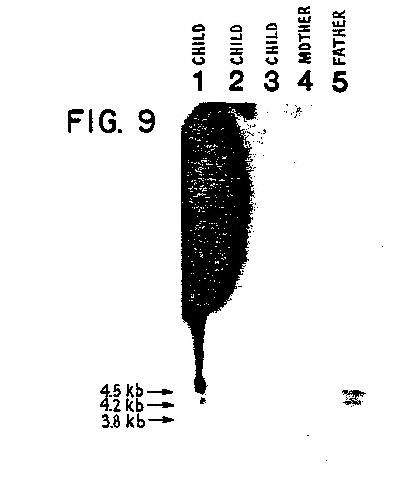

Fig. 9 is an autoradiograph of five individuals of the sixth family. The father's pattern appears in Lane 5, the mother's in Lane 4 and the three children in Lanes 1—3. In this family, disease susceptibility appeared to be linked to the 4.5 kb *Bg/*II fragment. Here, the affected father has a 4.5 kb fragment (haplotype A) and 4.2 kb fragment (haplotype B) and the mother has a 4.5 kb fragment (haplotype D) and a 3.8 kb fragment (haplotype C). Of the three children, only one has IDDM (Lane 3) and he inherited the 4.5 kb fragment from the father and the 3.8 kb fragment from the mother. Therefore, in this family the 4.5 kb *Bg/*II fragment is linked to the disease susceptibility allele on the paternal haplotype A.

In summary, five of the six probands in this set of diabetes families had at least one copy of the 4.2 kb *Bg/*III fragment. In a sample of unrelated control individuals, 4/16 individual DNA samples had the 4.2 kb fragment, suggesting an increased relative risk (RR) for IDDM associated with the 4.2 kb fragment due, presumably, to linkage disequilibrium between the polymorphic *Bg/*II site and a linked IDDM susceptibility allele.

$$\text{RR (relative risk} = \frac{(.834) \times (.75)}{(.25 \ (.167)} = 14$$

By comparison, the relative risk for the serologically defined HLA—DR3 = 3.3 and for HLA—DR4 = 6.4. By using more restriction endonucleases and more probes, it should be possible to generate a significantly higher value than the one indicated by these data with the HLA-DRα probe and *Bg/*II.

**Claims for the Contracting States: BE CH DE FR GB IT LI LU NL SE**

1. An HLA typing method based on HLA DNA restriction fragment length polymorphism characterized by the following steps:

(a) digesting genomic HLA DNA from an individual with a restriction endonuclease that produces a polymorphic digestion pattern of a Class II HLA DNA locus;

(b) subjecting the digest of (a) to genomic blotting using a labeled cDNA probe that is complementary to a Class II HLA DNA locus sequence involved in the polymorphism; and

(c) comparing the genomic blotting pattern obtained in (b) with a standard genomic blotting pattern for said HLA DNA sequence obtained using said restriction endonuclease and the same labeled cDNA probe or one having the same specificity.

2. The method of Claim 1 further characterized in that the locus is the DRα locus, a DRα related locus, or the DRβ locus.

3. The method of Claim 1 or 2 further characterized in that:

(i) the locus is the DRα locus and the restriction endonuclease is *Bg/*II or *Eco*RV, or

(ii) the locus is a DRα related locus and the restriction endonuclease is *Bg/*II or,

(iii) the locus is the DRβ locus or a DRβ related locus and the restriction endonuclease is *Eco*RI or *Bg/*II.

4. A method for determining the paternity of an individual based on HLA DNA restriction fragment length polymorphism characterized by the following steps:

(a) digesting genomic HLA DNA of the mother of the individual, the suspected father of the individual, and the individual with a restriction endonuclease that produces a polymorphic digestion pattern of a Class II DNA locus.

(b) subjecting each of the digests of (a) to genomic blotting using a labeled cDNA probe that is complementary to a Class II HLA DNA sequence involved in the polymorphism; and

(c) comparing the genomic blotting patterns obtained in (b) to determine correspondence between the individual's pattern and the mother's pattern and suspected father's pattern and thereby determining whether the suspected father is the actual father of the individual.

5. A method for determining transplant or transfusion compatibility based on HLA DNA restriction fragment length polymorphism characterized by the following steps:

(a) digesting genomic HLA DNA of the transplant or transfusion donor and the transplant or transfusion host with a restriction endonuclease that produces a polymorphic digestion pattern of a Class II HLA DNA locus;

(b) subjecting each of the digests of (a) to genomic blotting using a labeled cDNA probe that is complementary to a Class II HLA DNA sequence involved in the polymorphism; and

(c) comparing the genomic blotting patterns obtained in (b) to determine correspondence therebetween and thereby determining whether the transplant or transfusion donor's HLA is compatible with the transplant or transfusion host's HLA.

6. A method of determining whether an individual is susceptible to or has a disease characterized by the following steps:

(a) digesting genomic HLA DNA from an individual with a restriction endonuclease that produces a polymorphic digestion pattern of a Class II HLA locus that is associated with the disease;

(b) subjecting the digest of (a) to genomic blotting using a labeled cDNA probe that is complementary to the DNA sequence of said Class II HLA locus;

(c) comparing the genomic blotting pattern obtained in (b) with a standard pattern of an individual having said disease obtained using said restriction endonuclease and the same labeled cDNA probe or one having the same specificity.

7. The method of claim 6 wherein the disease is insulin dependent diabetes mellitus.

8. An HLA cDNA probe characterised in that the probe is specific to a single HLA Class II locus and comprises a labeled DNA sequence that is complementary to the DNA sequence at said locus.

9. The probe of Claim 8, further characterised in that the locus is the DRα locus, a DRα related locus, the DRβ locus, or a ORβ related locus.

10. The probe of Claim 8 further characterised in that the locus is the DRα locus and the nucleotide sequence of the probe is

```
ATCATAGCTG TGCTGATGAG CGCTCAGGAA TCATGGGCTA TCAAAGAAGA
ACATGTGATC ATCCAGGCCG AGTTCTATCT GAATCCTGAC CAATCAGGCG
AGTTTATGTT TGACTTTGAT GGTGATGAGA TTTTCCATGT GGATATGGCA
AAGAAGGAGA CGGTCTGGCG GCTTGAAGAA TTTGGACGAT TTGCCAGCTT
TGAGGCTCAA GGTGCATTGG CCAACATAGC TGTGGACAAA GCCAACCTGG
AAATCATGAC AAAGCGCTCC AACTATACTC CGATCACCAA TGTACCTCCA
GAGGTAACTG TGCTCACGAA CAGCCCTGTG GAACTGAGAG AGCCCAACGT
CCTCATCTGT TTCATCGACA AGTTCACCCC ACCAGTGGTC AATGTCACGT
GGCTTCGAAA TGGAAAACCT GTCACCACAG GAGTGTCAGA GACAGTCTTC
CTGCCCAGGG AAGACCACCT TTTCCGCAAG TTCCACTATC TCCCCTTCCT
GCCCTCAACT GAGGACGTTT ACGACTGCAG GGTGGAGCAC TGNGGCTTGG
ATGAGCCTCT TCTCAAGCAC TGGGAGTTTG ATGCTCCAAG CCCTCTCCCA
GAGACTACAG AGAACGTGGT GTGTGCCCTG GGCCTGACTG TGGGTCTGGT
GGGCATCATT ATTGGGACCA TCTTCATCAT CAAGGGAGTG CGCAAAAGCA
ATGCAGCAGA ACGCAGGGGG CCTCTGTAAG GCACATGGAG GTGATGATGT
TTCTTAGAGA GAAGATCACT GAAGAAACTT CTGCTTTAAT GACTTTACAA
AGCTGGCAAT ATTACAATCC TTGACCTCAG TGAAAGCAGT CATCTTCAGC
GTTTTCCAGC CCTATAGCCA CCCCAAGTGT GGTTATGCCT CCTCGATTGC
TCCGTACTCT AACATCTAGC TGGCTTCCCT GTCTATTGCC TTTTCCTGTA
TCTATTTTCC TCTATTTCCT ATCATTTTAT TATCACCATG CAATGCCTCT
GGAATAAAAC ATACAGGAGT CTGTCTCTGC TATGGAATGC CCCATGGGGC
TCTCTTGTGT ACTTATTGTT TAAGGTTTCC TCAAACTGTG ATTTTTCTG
```

wherein N is an unidentified nucleotide.

11. The probe of Claim 8 wherein the locus is DRα related and the probe contains the following subsequences

(i)

```
TNTGAACNCCAGCTGCCCTACAAACTCCATCTCAGCTTTTCTTCTCACTTCATG
TNAAAACTACTCCAGTGGCTGACTNAATTGCTGACCCTTCAAGCTCTGTCCTTA
TCCATTACCTCAAAGCAGTCATTCCTTAGTNAAGTTTCCAAC
```

( ii )

CACGGGAGNCCCAAGAGCCAACCAGACGCCTGAGACAACGGAGACTGTGCTCTG

TGCCCTGGGCCTGGTGCTGGGCCTAGTCGGCATCATCGTGGGCACCGTCCTNAT

CATAAAGTCTCTGCGTTCTGGCCATGACCCCC

( iii )

CACATTGACGAGTTCTTCCCACCAGTCCTCAANGTCACGTGGGCCGCGCAACGG

GGAGCTGGTCACTGAGGG and

( iv )

AAGGAGACCGTCTGGCATCTGGAGGAGTTTGGCCAAGCCTTTTCCCTTTGAGGC

TCAGGGCGGGCTGGCTAACATTGCTATATTGAACAACAACTTGAAACCTTGA

wherein N is an unidentified nucleotide.

12. An HLA cDNA sequence characterized in that the sequence is complementary to a single HLA Class II locus.

13. The cDNA sequence of claim 12, further characterized in that the HLA locus is the DRα locus and the sequence is

```
ATCATAGCTG  TGCTGATGAG  CGCTCAGGAA  TCATGGGCTA  TCAAAGAAGA
ACATGTGATC  ATCCAGGCCG  AGTTCTATCT  GAATCCTGAC  CAATCAGGCG
AGTTTATGTT  TGACTTTGAT  GGTGATGAGA  TTTTCCATGT  GGATATGGCA
AAGAAGGAGA  CGGTCTGGCG  GCTTGAAGAA  TTTGGACGAT  TTGCCAGCTT
TGAGGCTCAA  GGTGCATTGG  CCAACATAGC  TGTGGACAAA  GCCAACCTGG
AAATCATGAC  AAAGCGCTCC  AACTATACTC  CGATCACCAA  TGTACCTCCA
GAGGTAACTG  TGCTCACGAA  CAGCCCTGTG  GAACTGAGAG  AGCCCAACGT
CCTCATCTGT  TTCATCGACA  AGTTCACCCC  ACCAGTGGTC  AATGTCACGT
GGCTTCGAAA  TGGAAAACCT  GTCACCACAG  GAGTGTCAGA  GACAGTCTTC
CTGCCCAGGG  AAGACCACCT  TTTCCGCAAG  TTCCACTATC  TCCCCTTCCT
GCCCTCAACT  GAGGACGTTT  ACGACTGCAG  GGTGGAGCAC  TGNGGCTTGG
ATGAGCCTCT  TCTCAAGCAC  TGGGAGTTTG  ATGCTCCAAG  CCCTCTCCCA
GAGACTACAG  AGAACGTGGT  GTGTGCCCTG  GGCCTGACTG  TGGGTCTGGT
GGGCATCATT  ATTGGGACCA  TCTTCATCAT  CAAGGGAGTG  CGCAAAAGCA
ATGCAGCAGA  ACGCAGGGGG  CCTCTGTAAG  GCACATGGAG  GTGATGATGT
TTCTTAGAGA  GAAGATCACT  GAAGAAACTT  CTGCTTTAAT  GACTTTACAA
AGCTGGCAAT  ATTACAATCC  TTGACCTCAG  TGAAAGCAGT  CATCTTCAGC
GTTTTCCAGC  CCTATAGCCA  CCCCAAGTGT  GGTTATGCCT  CCTCGATTGC
TCCGTACTCT  AACATCTAGC  TGGCTTCCCT  GTCTATTGCC  TTTTCCTGTA
TCTATTTTCC  TCTATTTCCT  ATCATTTTAT  TATCACCATG  CAATGCCTCT
GGAATAAAAC  ATACAGGAGT  CTGTCTCTGC  TATGGAATGC  CCCATGGGGC
TCTCTTGTGT  ACTTATTGTT  TAAGGTTTCC  TCAAACTGTG  ATTTTTCTG
```

wherein N is an unidentified nucleotide.

# EP 0 084 796 B1

**Claims for the Contracting State: AT**

1. An HLA typing method based on HLA DNA restriction fragment length polymorphism characterized by the following steps:

(a) digesting genomic HLA DNA from an individual with a restriction endonuclease that produces a polymorphic digestion pattern of a Class II HLA DNA locus;

(b) subjecting the digest of (a) to genomic blotting or using a labeled cDNA probe that is complementary to a Class II HLA DNA locus sequence involved in the polymorphism; and

(c) comparing the genomic blotting pattern obtained in (b) with a standard genomic blotting pattern for said HLA DNA sequence obtained using said restriction endonuclease and the same labeled cDNA probe or one having the same specificity.

2. The method of Claim 1 further characterized in that the locus is the DRα locus, a DRα related locus, or the DRβ locus.

3. The method of Claim 1 or 2 further characterized in that:

(i) the locus is the DRα locus and the restriction endonuclease is *Bg*/II or *Eco*RV, or

(ii) the locus is a DRα related locus and the restriction endonuclease is *Bg*/II or,

(iii) the locus is the DRβ locus or a DRβ related locus and the restriction endonuclease is *Eco*RI or *Bg*/II.

4. A method for determining the paternity of an individual based on HLA DNA restriction fragment length polymorphism characterized by the following steps:

(a) digesting genomic HLA DNA of the mother of the individual, the suspected father of the individual, and the individual with a restriction endonuclease that produces a polymorphic digestion pattern of a Class II HLA DNA locus;

(b) subjecting each of the digests of (a) to genomic blotting using a labeled cDNA probe that is complementary to a Class II HLA DNA sequence involved in the polymorphism; and

(c) comparing the genomic blotting patterns obtained in (b) to determine correspondence between the individual's pattern and the mother's pattern and suspected father's pattern and thereby determining whether the suspected father is the actual father of the individual.

5. A method for determining transplant or transfusion compatibility based on HLA DNA restriction fragment length polymorphism characterized by the following steps:

(a) digesting genomic HLA DNA of the transplant or transfusion donor and the transplant or transfusion host with a restriction endonuclease that produces a polymorphic digestion pattern of a Class II HLA DNA locus;

(b) subjecting each of the digests of (a) to genomic blotting using a labeled cDNA probe that is complementary to a Class II HLA DNA sequence involved in the polymorphism; and

(c) comparing the genomic blotting patterns obtained in (b) to determine correspondence therebetween and thereby determining whether the transplant or transfusion donor's HLA is compatible with the transplant or transfusion host's HLA.

6. A method of determining whether an individual is susceptible to or has a disease characterized by the following steps:

(a) digesting genomic HLA DNA from the individual with a restriction endonuclease that produces a polymorphic digestion pattern of a Class II HLA locus that is associated with the disease;

(b) subjecting the digest of (a) to genomic blotting using a labeled cDNA probe that is complementary to the DNA sequence of said Class II HLA;

(c) comparing the genomic blotting pattern obtained in (b) with a standard pattern of an individual having said disease obtained using said restriction endonuclease and the same labeled cDNA probe or one having the same specificity.

7. The method of claim 6 wherein the disease is insulin dependent diabetes mellitus.

**Patentansprüche für die Vertragsstaaten: BE CH DE FR GB IT LI LU NL SE**

1. HLA-Charakterisierungsverfahren auf Basis des Längenpolymorphismus von HNA-DNS-Restriktionsfragmenten, gekennzeichnet durch die folgenden Schritte:

(a) Verdauen von Genom-HLA-DNS eines individuums mit einer Restriktions-Endonuclease, das ein polymorphes Verdauungsmuster eines Klasse II-HLA-DNS-Locus erzeugt;

(b) Unterwerfen des Verdauungsprodukts aus (a) dem Genom-Blotting unter Verwendung einer markierten cDNS-Sonde, die komplementär zur Sequenz des in den Polymorphismus eingebezogenen Klasse II-HLA-DNS-Locus ist; und

(c) Vergleichen desin (b) erhaltenen Genom-Blottingmusters mit einem Standard-Genom-Blottingmusters für diese HLN-DNS-Sequenz, das unter Verwendung dieser Restriktions-Endonuclease und das gleichen markierten cDNA-Sonde oder eine mit der gleichen Spezifität erhalten wurde.

2. Verfahren nach Anspruch 1, weiterhin dadurch gekennzeichnet, daß der Locus der DRα-Locus, ein DRα-bezogener Locus oder der DRβ-Locus ist.

3. Verfahren nach Anspruch 1 oder 2, weiterhin dadurch gekennzeichnet, daß:

(i) der Locus der DRα-Locus ist und die die Retriktions-Endonclease *Bg*/II oder *Eco*RV ist, oder

(ii) der Locus ein DRα-bezogener Locus ist und die Restriktions-Endonuclease *Bg*/II ist, oder

(iii) der Locus der DRβ-Locus oder ein DRβ-bezogener Locus ist und die Restriktions-Endonuclease *Eco*RI oder *Bg*/II ist.

4. Verfahren zur Bestimmung der Vatershaft eines Individuums auf Basis des Längenpolymorphismus von HLA-DNS-Restriktionsfragmenten, gekennzeichnet durch die folgenden Schritte:

(a) Verdauen von Genom-HLA-DNS der Mutter oder des Individuums, des verdähtigten Vaters des Individuums und des Individuums mit einer Restriktions-Endonuclease, die ein polymorphes Verdauungsmuster eines Klasse II-DNS-Locus erzeugt;

(b) Unterwerden eines jeden der Verdauungsprodukte aus (a) dem Genom-Blotting unter Verwendung eier markierten cDNS-Sonde, die komplementär zu einer in den Polymorphismus einbezogenen Sequenz der Klasse II-HLA-DNS ist; und

(c) Vergleichen des in (b) erhaltenen Genom-Blottingmusters zur Bestimmung von Übereinstimmungen zwischen dem Muster des Individuums und den Mustern der Mutter und des verdächtigten Vaters und dadurch Bestimmen, ob der verdächtige Vater der tatsächliche Vater des Individuums ist.

5. Verfahren zur Bestimmung einer Transplantat- oder Transfusionsverträglichkeit auf Basis des Längenpolymorphismus von HLA-DNS-Restriktionsfragmenten, gekennzeichnet durch die folgenden Schritte:

(a) Verdauen von Genom-HLA-DNS des Transplantat- oder Transfusionsspenders und des Transplantat- oder Transfusionsempfängers mit einer Restriktions-Endonuclease, die ein polymorphes Verdauungsmuster eines Klasse II-HLA-DNS-Locus erzeugt;

(b) Unterwerfen eines jeden Verdauungsprodukts aus (a) dem Genom-Blotting unter Verwendung einer markierten cDNA-Sonde, die komplementär zu einer in den Polymorphismus einbezogenen Sequenz einer Klasse II-HLA-DNS ist; und

(c) Vergleichen des in (b) erhaltenen Genom-Blottingmusters zur Bestimmung von dazwischen bestehenden Übereinstimmungen und dadurch Bestimmen, ob die HLA des Transplantat- oder Transfusionnspenders mit der HLA des Transplantat- oder Transfusionsempfängers vereinbar ist.

6. Verfahren zur Bestimmung, ob ein Individuum eine Krankheit hat oder für diese empfänglich ist, gekennzeichnet durch die folgenden Schritte:

(a) Verdauen von Genom-HLA-DNS des Individuums mit einer Restriktions-Endonuclease, die ein polymorphes Verdauungsmuster eines Klasse II-HLA-Locus, der mit der Krankheit verbunden ist, erzeugt;

(b) Unterwerfen des Verdauungsprodukts aus (a) dem Genom-Blotting unter Verwendung einer markierten cDNS-Sonde, die komplementär zur DNS-Sequenz dieses Klasse II-HLA-Locus ist;

(c) Vergleichen des in (b) erhaltenen Genom-Blottingmusters mit einem Standardmuster eines Individuums, das diese Krankheit hat, das unter Verwendung dieser Restriktions-Endonuclease und der gleichen markierten cDNS-Sonde oder einer mit der gleichen Spezifität erhalten wurde.

7. Verfahren nach Anspruch 6, worin die Krankheit Insulin-abhängier Diabetes mellitus ist.

8. HLA-cDNS-Sonde, dadurch gekennzeichnet, daß die Sonde spezifisch für einen einzelnen HLA-Klasse II-Locus ist und eine markierte DNS-Sequenz umfaßt, die komplementär zur DNS-Sequenz dieses Locus ist.

9. Sonde nach Anspruch 8, weiterhin dadurch gekennzeichnet daß der Locus der DRα-Locus, ein DRα-bezogener Locus, der DRβ-Locus oder ein DRβ-bezogener Locus ist.

10. Sonde nach Anspruch 8, weiterhin dadurch gekennzeichnet, daß der Locus der DRα-Locus ist und die Nucleotidsequenz der Probe

```
ATCATAGCTG  TGCTGATGAG  CGCTCAGGAA  TCATGGGCTA  TCAAAGAAGA

ACATGTGATC  ATCCAGGCCG  AGTTCTATCT  GAATCCTGAC  CAATCAGGCG

AGTTTATGTT  TGACTTTGAT  GGTGATGAGA  TTTTCCATGT  GGATATGGCA

AAGAAGGAGA  CGGTCTGGCG  GCTTGAAGAA  TTTGGACGAT  TTGCCAGCTT

TGAGGCTCAA  GGTGCATTGG  CCAACATAGC  TGTGGACAAA  GCCAACCTGG

AAATCATGAC  AAAGCGCTCC  AACTATACTC  CGATCACCAA  TGTACCTCCA

GAGGTAACTG  TGCTCACGAA  CAGCCCTGTG  GAACTGAGAG  AGCCCAACGT

CCTCATCTGT  TTCATCGACA  AGTTCACCCC  ACCAGTGGTC  AATGTCACGT

GGCTTCGAAA  TGGAAAACCT  GTCACCACAG  GAGTGTCAGA  GACAGTCTTC

CTGCCCAGGG  AAGACCACCT  TTTCCGCAAG  TTCCACTATC  TCCCCTTCCT

GCCCTCAACT  GAGGACGTTT  ACGACTGCAG  GGTGGAGCAC  TGNGGCTTGG

ATGAGCCTCT  TCTCAAGCAC  TGGGAGTTTG  ATGCTCCAAG  CCCTCTCCCA
```

GAGACTACAG AGAACGTGGT GTGTGCCCTG GGCCTGACTG TGGGTCTGGT

GGGCATCATT ATTGGGACCA TCTTCATCAT CAAGGGAGTG CGCAAAAGCA

ATGCAGCAGA ACGCAGGGGG CCTCTGTAAG GCACATGGAG GTGATGATGT

TTCTTAGAGA GAAGATCACT GAAGAAACTT CTGCTTTAAT GACTTTACAA

AGCTGGCAAT ATTACAATCC TTGACCTCAG TGAAAGCAGT CATCTTCAGC

GTTTTCCAGC CCTATAGCCA CCCCAAGTGT GGTTATGCCT CCTCGATTGC

TCCGTACTCT AACATCTAGC TGGCTTCCCT GTCTATTGCC TTTTCCTGTA

TCTATTTTCC TCTATTTCCT ATCATTTTAT TATCACCATG CAATGCCTCT

GGAATAAAAC ATACAGGAGT CTGTCTCTGC TATGGAATGC CCCATGGGGC

TCTCTTGTGT ACTTATTGTT TAAGGTTTCC TCAAACTGTG ATTTTTCTG

ist, worin N ein nicht identifiziertes Nucleotid ist.

11. Sonde nach Anspruch 8, worin der Locus DRα-bezogen ist und die Sonde die folgenden Untersequenzen enthält

(i)

TNTGAACNCCAGCTGCCCTACAAACTCCATCTCAGCTTTTCTTCTCACTTCATG

TNAAAACTACTCCAGTGGCTGACTNAATTGCTGACCCTTCAAGCTCTGTCCTTA

TCCATTACCTCAAAGCAGTCATTCCTTAGTNAAGTTTCCAAC

(ii)

CACGGGAGNCCCAAGAGCCAACCAGACGCCTGAGACAACGGAGACTGTGCTCTG

TGCCCTGGGCCTGGTGCTGGGCCTAGTCGGCATCATCGTGGGCACCGTCCTNAT

CATAAAGTCTCTGCGTTCTGGCCATGACCCCC

(iii)

CACATTGACGAGTTCTTCCCACCAGTCCTCAANGTCACGTGGGCCGCGCAACGG

GGAGCTGGTCACTGAGGG    und

(iv)

AAGGAGACCGTCTGGCATCTGGAGGAGTTTGGCCAAGCCTTTTCCCTTTGAGGC

TCAGGGCGGGCTGGCTAACATTGCTATATTGAACAACAACTTGAAACCTTGA

worin N ein nicht identifiziertes Nucleotid ist.

12. HLA-cDNS-Sequenz, dadurch gekennzeichnet, daß die Sequenz komplementär zum einem einzelnen HLA-Klasse II-Locus ist.

13. cDNS-Sequenz nach Anspruch 12, weiterhin dadurch gekennzeichnet, daß der HLA-Locus der DRα-Locus ist und die Sequenz

ATCATAGCTG TGCTGATGAG CGCTCAGGAA TCATGGGCTA TCAAAGAAGA

ACATGTGATC ATCCAGGCCG AGTTCTATCT GAATCCTGAC CAATCAGGCG

AGTTTATGTT TGACTTTGAT GGTGATGAGA TTTTCCATGT GGATATGGCA

AAGAAGGAGA CGGTCTGGCG GCTTGAAGAA TTTGGACGAT TTGCCAGCTT

TGAGGCTCAA GGTGCATTGG CCAACATAGC TGTGGACAAA GCCAACCTGG

AAATCATGAC AAAGCGCTCC AACTATACTC CGATCACCAA TGTACCTCCA

GAGGTAACTG TGCTCACGAA CAGCCCTGTG GAACTGAGAG AGCCCAACGT

CCTCATCTGT TTCATCGACA AGTTCACCCC ACCAGTGGTC AATGTCACGT

GGCTTCGAAA TGGAAAACCT GTCACCACAG GAGTGTCAGA GACAGTCTTC

CTGCCCAGGG AAGACCACCT TTTCCGCAAG TTCCACTATC TCCCCTTCCT

GCCCTCAACT GAGGACGTTT ACGACTGCAG GGTGGAGCAC TGNGGCTTGG

ATGAGCCTCT TCTCAAGCAC TGGGAGTTTG ATGCTCCAAG CCCTCTCCCA

GAGACTACAG AGAACGTGGT GTGTGCCCTG GGCCTGACTG TGGGTCTGGT

GGGCATCATT ATTGGGACCA TCTTCATCAT CAAGGGAGTG CGCAAAAGCA

ATGCAGCAGA ACGCAGGGGG CCTCTGTAAG GCACATGGAG GTGATGATGT

TTCTTAGAGA GAAGATCACT GAAGAAACTT CTGCTTTAAT GACTTTACAA

AGCTGGCAAT ATTACAATCC TTGACCTCAG TGAAAGCAGT CATCTTCAGC

GTTTTCCAGC CCTATAGCCA CCCCAAGTGT GGTTATGCCT CCTCGATTGC

TCCGTACTCT AACATCTAGC TGGCTTCCCT GTCTATTGCC TTTTCCTGTA

TCTATTTTCC TCTATTTCCT ATCATTTTAT TATCACCATG CAATGCCTCT

GGAATAAAAC ATACAGGAGT CTGTCTCTGC TATGGAATGC CCCATGGGGC

TCTCTTGTGT ACTTATTGTT TAAGGTTTCC TCAAACTGTG ATTTTTCTG

worin N ein nicht identifiziertes Nucleotid ist.

**Patentansprüche für den Vertragsstaat: AT**

1. HLA-Charakterisierungsverfahren auf Basis des Längenpolymorphismus von HNA-DNS-Restriktionsfragmenten, gekennzeichnet durch die folgenden Schritte:

(a) Verdauen von Genom-HLA-DNS eines individuums mit einer Restriktions-Endonucleas, das ein polymorphes Verdauungsmuster eines Klasse II-HLA-DNS-Locus erzeugt;

(b) Unterwerfen des Verdauungsprodukts aus (a) dem Genom-Blotting unter Verwendung einer markierten cDNS-Sonde, die komplementär zur Sequenz des in den Polymorphismus eingebezogenen Klasse II-HLA-DNS-Locus ist; und

(c) Vergleichen desin (b) erhaltenen Genom-Blottingmusters mit einem Standard-Genom-Blottingmusters für diese HLN-DNS-Sequenz, das unter Verwendung dieser Restriktions-Endonuclease und der gleichen markierten cDNA-Sonde oder eine mit der gleichen Spezifität erhalten wurde.

2. Verfahren nach Anspruch 1, weiterhin dadurch gekennzeichnet, daß der Locus der DRα-Locus, ein DRα-bezogener Locus oder der DRβ-Locus ist.

3. Verfahren nach Anspruch 1 oder 2, weiterhin dadurch gekennzeichnet, daß:

(i) der Locus der DRα-Locus ist und die die Retriktions-Endonclease BglII oder EcoRV ist, oder

(ii) der Locus ein DRα-bezogener Locus ist und die Restriktions-Endonuclease BglII ist, oder

(iii) der Locus der DRβ-Locus oder ein DRβ-bezogener Locus ist und die Restriktions-Endonuclease EcoRI oder BglII ist.

4. Verfahren zur Bestimmung der Vatershaft eines Individuums auf Basis des Längenpolymorphismus von HLA-DNS-Restriktionsfragmenten, gekennzeichnet durch die folgenden Schritte:

(a) Verdauen von Genom-HLA-DNS der Mutter oder des Individuums, des verdähtigten Vaters des Individuums und des Individuums mit einer Restriktions-Endonuclease, die ein polymorphes Verdauungsmuster eines Klasse II-DNS-Locus erzeugt;

(b) Unterwerden eines jeden der Verdauungsprodukte aus (a) dem Genom-Blotting unter Verwendung eier markierten cDNS-Sonde, die komplementär zu einer in den Polymorphismus einbezogenen Sequenz der Klasse II-HLA-DNS ist; und

(c) Vergleichen des in (b) erhaltenen Genom-Blottingmusters zur Bestimmung von Übereinstimmungen zwischen dem Muster des Individuums und den Mustern der Mutter und des verdächtigten Vaters und dadurch Bestimmen, ob der verdächtige Vater der tatsächliche Vater des Individuums ist.

5. Verfahren zur Bestimmung einer Transplantat- oder Transfusionsverträglichkeit auf Basis des Längenpolymorphismus von HLA-DNS-Restriktionsfragmenten, gekennzeichnet durch die folgenden Schritte:

(a) Verdauen von Genom-HLA-DNS des Transplantat- oder Transfusionsspenders und des Transplantat- oder Transfusionsempfängers mit einer Restriktions-Endonuclease, die ein polymorphes Verdauungsmuster eines klasse II-HLA-DNS-Locus erzeugt;

(b) Unterwerfen des Verdauungsprodukts aus (a) dem Genom-Blotting unter Verwendung einer markierten cDNS-Sonde, die komplementär zur DNS-Sequenz dieses Klasse II-HLA-Locus ist;

# EP 0 084 796 B1

(c) Vergleichen des in (b) erhaltenen Genom-Blottingmusters zur Bestimmung von dazwischen bestehenden Übereinstimmungen und dadurch Bestimmen, ob die HLA des Transplantat- oder Transfusionsspenders mit der HLA des Transplantat- oder Transfusions empfängers vereinbar ist.

6. Verfahren zur Bestimmung, ob ein Individuum eine Krankheit hat oder für diese empfänglich ist, gekennzeichnet durch die folgenden Schritte:

(a) Verdauen von Genom-HLA-DNS des Individuums mit einer Restriktions-Endonuclease, die ein polymorphes Verdauungsmuster eines Klasse II-HLA-Locus, der mit der Krankheit verbunden ist, erzeugt;

(b) Unterwerfen des Verdauungsprodukts aus (a) dem Genom-Blotting unter Verwendung einer markierten cDNS-Sonde, die komplementär zur DNS-Sequenz dieses Klasse II-HLA-Locus ist;

(c) Vergleichen des in (b) erhaltenen Genom-Blottingmusters mit einem Standardmuster eines Individuums, das diese Krankheit hat, das unter Verwendung dieser Restriktions-Endonuclease und der gleichen markierten cDNS-Sonde oder einer mit der gleichen Spezifität erhalten wurde.

7. Verfahren nach Anspruch 6, worin die Krankheit Insulin-abhängiger Diabetes mellitus ist.

**Revendications pour les Etats contractants: BE CH DE FR GB IT LI LU NL SE**

1. Méthode de typage de HLA basée sur le polymorphisme de la longueur des fragments de restriction d'ADN de HLA, caractérisée par les étapes suivantes:

(a) digérer l'ADN de HLA génomique provenant d'un individu avec une endonucléase de restriction qui produit un profil de digestion polymorphe d'un locus ADN de HLA Classe II;

(b) soumettre le produit de digestion de (a) au transfer ("blotting") génomique en utilisant une sonde ADNc marquée qui est complémentaire d'une séquence de locus ADN de HLA Classe II impliquée dans le polymorphisme; et

(c) comparer le profil du transfert ("blotting") génomique obtenu en (b) avec un profil de transfert génomique standard pour ladite séquence d'ADN de HLA obtenue en utilisant ladite endonucléase de restriction et la même sonde ADNc marquée ou une sonde ayant la même spécificité.

2. La méthode de la Revendication 1, caractérisé encore en ce que le locus est le locus DRα, un locus lié à DRα ou le locus DRβ.

3. La méthode de la Revendication 1 ou 2, caractérisée en ce que:

(i) le locus est le locus DRα et l'endonucléase de restriction est *Bg/*II ou *Eco*RV, ou

(ii) le locus est un locus lié à DRα et l'endonucléase de restriction est *Bg/*II, ou

(iii) le locus est le locus DRβ ou un locus lié à DRβ et l'endonucléase de restriction est *Eco*RI ou *Bg/*II.

4. Méthode pour la détermination de la paternité d'un individu basée sur le polymorphisme de la longueur des fragments de restriction d'ADN de HLA, caractérisée par les étapes suivantes:

(a) digérer l'ADN de HLA génomique de la mère de l'individu, du père suspecté de l'individu et de l'individu avec une endonucléase de restriction qui produit un profil de digestion polymorphe d'un locus ADN de Classe II,

(b) soumettre chcun des produits de digestion de (a) au transfert ("blotting") génomique en utilisant une sonde ADNc marquée qui est complémentaire d'une séquence d'ADN de HLA Classe II impliquée dans le polymorphisme; et

(c) comparer les profils du transfert génomique obtenus en (b) pour déterminer la correspondance entre le profil de l'individu et le profil de la mère ainsi que le profil du père suspecté et établir ainsi sil le père suspecté est le père réel de l'individu.

5. méthode pour déterminer la compatibilité d'un greffon ou d'une transfusion, basée sur le polymorphisme de la longueur des fragments de restriction d'ADN de HLA, caractérisée par les étapes suivantes:

(a) digérer l'ADN de HLA génomique du donneur du greffon ou de la transfusion et du receveur du greffon ou de la transfusion avec une endonucléase de restriction qui produit un profil de digestion polymorphe d'un locus ADN de HLA Classe II;

(b) soumettre chacun de produits de digestion de (a) au transfert ("blotting") génomique en utilisant une sonde ADNc marquée qui est complémentaire d'une séquence d'ADN de HLA Clase II impliquée dans le polymorphisme; et

(c) comparer les profils du transfert génomique obtenus en (b) pour déterminer la correspondance entre eux et établir ainsi si le HLA du donneur du greffon ou de la transfusion est compatible avec le HLA du receveur du greffon ou de la transfusion.

6. Méthode pour déterminer si un individu est sensible à une maladie ou en est atteint, caractérisée par les étapes suivantes:

(a) digérer l'ADN du HLA génomique de l'individu avec endonucléase de restriction qui produit un profil de digestion polymorphe d'un locus HLA Classe II qui est associé à la maladie;

(b) soumettre le produit de digestion de (a) au transfert ("blotting") génomique en utilisant une sonde ADNc marquée qui est complémentaire de la séquence d'ADN dudit locus HLA Classe II;

(c) comparer le profil du transfert génomique obtenu en (b) avec un profil standard d'un individu atteint de ladite maladie, obtenu en utilisant ladite endonucléase de restriction et la même sonde ADNc marquée ou une sonde ayant la même.

7. La méthode de la Revendication 6 dans laquelle la maladie est le diabète insulinodépendant.

19

8. Sonde ADNc de HLA caractérisée en ce que la sonde est spécifique pour un seul locus HLA Classe II et comprend une séquence d'ADN marquée est complémentaire de la séquence d'ADN audit locus.

9. La sonde de la Revendication 8, caractérisée en outre en ce que le locus est le locus DRα, un locus lié à DRα ou un locus lié à DRβ.

10. La sonde de la Revendication 8, caractérisée en outre en ce que le locus est le locus DRα et la séquence nucléotidique de la sonde est

```
ATCATAGCTG TGCTGATGAG CGCTCAGGAA TCATGGGCTA TCAAAGAAGA
ACATGTGATC ATCCAGGCCG AGTTCTATCT GAATCCTGAC CAATCAGGCG
AGTTTATGTT TGACTTTGAT GGTGATGAGA TTTTCCATGT GGATATGGCA
AAGAAGGAGA CGGTCTGGCG GCTTGAAGAA TTTGGACGAT TTGCCAGCTT
TGAGGCTCAA GGTGCATTGG CCAACATAGC TGTGGACAAA GCCAACCTGG
AAATCATGAC AAAGCGCTCC AACTATACTC CGATCACCAA TGTACCTCCA
GAGGTAACTG TGCTCACGAA CAGCCCTGTG GAACTGAGAG AGCCCAACGT
CCTCATCTGT TTCATCGACA AGTTCACCCC ACCAGTGGTC AATGTCACGT
GGCTTCGAAA TGGAAAACCT GTCACCACAG GAGTGTCAGA GACAGTCTTC
CTGCCCAGGG AAGACCACCT TTTCCGCAAG TTCCACTATC TCCCCTTCCT
GCCCTCAACT GAGGACGTTT ACGACTGCAG GGTGGAGCAC TGNGGCTTGG
ATGAGCCTCT TCTCAAGCAC TGGGAGTTTG ATGCTCCAAG CCCTCTCCCA
GAGACTACAG AGAACGTGGT GTGTGCCCTG GGCCTGACTG TGGGTCTGGT
GGGCATCATT ATTGGGACCA TCTTCATCAT CAAGGGAGTG CGCAAAAGCA
ATGCAGCAGA ACGCAGGGGG CCTCTGTAAG GCACATGGAG GTGATGATGT
TTCTTAGAGA GAAGATCACT GAAGAAACTT CTGCTTTAAT GACTTTACAA
AGCTGGCAAT ATTACAATCC TTGACCTCAG TGAAAGCAGT CATCTTCAGC
GTTTTCCAGC CCTATAGCCA CCCCAAGTGT GGTTATGCCT CCTCGATTGC
TCCGTACTCT AACATCTAGC TGGCTTCCCT GTCTATTGCC TTTTCCTGTA
TCTATTTTCC TCTATTTCCT ATCATTTTAT TATCACCATG CAATGCCTCT
GGAATAAAAC ATACAGGAGT CTGTCTCTGC TATGGAATGC CCCATGGGGC
TCTCTTGTGT ACTTATTGTT TAAGGTTTCC TCAAACTGTG ATTTTTCTG
```

ou N est un nucléotide non identifié.

11. La sonde de la Revendication 8 où le locus est lié a DRα et la sonde contient les séquences suivantes

( i )

```
TNTGAACNCCAGCTGCCCTACAAACTCCATCTCAGCTTTTCTTCTCACTTCATG
TNAAAACTACTCCAGTGGCTGACTNAATTGCTGACCCTTCAAGCTCTGTCCTTA
TCCATTACCTCAAAGCAGTCATTCCTTAGTNAAGTTTCCAAC
```

( ii )

```
CACGGGAGNCCCAAGAGCCAACCAGACGCCTGAGACAACGGAGACTGTGCTCTG
TGCCCTGGGCCTGGTGCTGGGCCTAGTCGGCATCATCGTGGGCACCGTCCTNAT
CATAAAGTCTCTGCGTTCTGGCCATGACCCCC
```

( iii )

```
CACATTGACGAGTTCTTCCCACCAGTCCTCAANGTCACGTGGGCCGCGCAACGG
GGAGCTGGTCACTGAGGG      et
```

(iv)

AAGGAGACCGTCTGGCATCTGGAGGAGTTTGGCCAAGCCTTTTCCCTTTGAGGC

TCAGGGCGGGCTGGCTAACATTGCTATATTGAACAACAACTTGAAACCTTGA

où N est un nucléotide non identifié.

12. Séquence d'ADNc de HLA caractérisée en ce que la séquence est complémentaire d'un seul locus HLA Classe II.

13. La séquence ADNc de la Revendication 12, caractérisée outre en ce que le locus HLA est le locus DRα et la séquence est

```
ATCATAGCTG  TGCTGATGAG  CGCTCAGGAA  TCATGGGCTA  TCAAAGAAGA
ACATGTGATC  ATCCAGGCCG  AGTTCTATCT  GAATCCTGAC  CAATCAGGCG
AGTTTATGTT  TGACTTTGAT  GGTGATGAGA  TTTTCCATGT  GGATATGGCA
AAGAAGGAGA  CGGTCTGGCG  GCTTGAAGAA  TTTGGACGAT  TTGCCAGCTT
TGAGGCTCAA  GGTGCATTGG  CCAACATAGC  TGTGGACAAA  GCCAACCTGG
AAATCATGAC  AAAGCGCTCC  AACTATACTC  CGATCACCAA  TGTACCTCCA
GAGGTAACTG  TGCTCACGAA  CAGCCCTGTG  GAACTGAGAG  AGCCCAACGT
CCTCATCTGT  TTCATCGACA  AGTTCACCCC  ACCAGTGGTC  AATGTCACGT
GGCTTCGAAA  TGGAAAACCT  GTCACCACAG  GAGTGTCAGA  GACAGTCTTC
CTGCCCAGGG  AAGACCACCT  TTTCCGCAAG  TTCCACTATC  TCCCCTTCCT
GCCCTCAACT  GAGGACGTTT  ACGACTGCAG  GGTGGAGCAC  TGNGGCTTGG
ATGAGCCTCT  TCTCAAGCAC  TGGGAGTTTG  ATGCTCCAAG  CCCTCTCCCA
GAGACTACAG  AGAACGTGGT  GTGTGCCCTG  GGCCTGACTG  TGGGTCTGGT
GGGCATCATT  ATTGGGACCA  TCTTCATCAT  CAAGGGAGTG  CGCAAAAGCA
ATGCAGCAGA  ACGCAGGGGG  CCTCTGTAAG  GCACATGGAG  GTGATGATGT
TTCTTAGAGA  GAAGATCACT  GAAGAAACTT  CTGCTTTAAT  GACTTTACAA
AGCTGGCAAT  ATTACAATCC  TTGACCTCAG  TGAAAGCAGT  CATCTTCAGC
GTTTTCCAGC  CCTATAGCCA  CCCCAAGTGT  GGTTATGCCT  CCTCGATTGC
TCCGTACTCT  AACATCTAGC  TGGCTTCCCT  GTCTATTGCC  TTTTCCTGTA
TCTATTTTCC  TCTATTTCCT  ATCATTTTAT  TATCACCATG  CAATGCCTCT
GGAATAAAAC  ATACAGGAGT  CTGTCTCTGC  TATGGAATGC  CCCATGGGGC
TCTCTTGTGT  ACTTATTGTT  TAAGGTTTCC  TCAAACTGTG  ATTTTTCTG
```

où N est un nucléotide non identifié.

**Revendications pour l'Etat contractant: AT**

1. Méthode de typage de HLA basée sur le polymorphisme de la longueur des fragments de restriction d'ADN de HLA, caractérisée par les étapes suivantes:

(a) digérer l'ADN de HLA génomique provenant d'un individu avec une endonucléase de restriction qui produit un profil de digestion polymorphe d'un locus ADN de HLA Classe II;

(b) soumettre le produit de digestion de (a) au transfert ("blotting") génomique en utilisant une sonde ADNc marquée qui est complémentaire d'une séquence de locus ADN de HLA Classe II impliquée dans le polymorphisme; et

(c) comparer le profil du transfert ("blotting") génomique obtenu en (b) avec un profil de transfert génomique standard pour ladite séquence d'ADN de HLA obtenue en utilisant ladite endonucléase de restriction et la même sonde ADNc marquée ou une sonde ayant la même spécificité.

**EP 0 084 796 B1**

2. La méthode de la Revendication 1, caractérisé encore en ce que le locus est le locus DRα, un locus lié à DRα ou le locus DRβ.

3. La méthode de la Revendication 1 ou 2, caractérisée en ce que:

(i) le locus est le locus DRα et l'endonucléase de restriction est *Bg/*II ou *Eco*RV, ou

(ii) le locus est un locus lié à DRα et l'endonucléase de restriction est *Bg/*II, ou

(iii) le locus est le locus DRβ ou un locus lié à DRβ et l'endonucléase de restriction est *Eco*RI ou *Bg/*II.

4. Méthode pour la détermination de la paternité d'un individu basée sur le polymorphisme de la longueur des fragments de restriction d'ADN de HLA, caractérisée par les étapes suivantes:

(a) digérer l'ADN de HLA génomique de la mère de l'individu, du père suspecté de l'individu et de l'individu avec une endonucléase de restriction qui produit un profil de digestion polymorphe d'un locus ADN de Classe II,

(b) soumettre chacun des produits de digestion de (a) au transfert ("blotting") génomique en utilisant une sonde ADNc marquée qui est complémentaire d'une séquence d'ADN de HLA Classe II impliquée dans le polymorphisme; et

(c) comparer les profils du transfert génomique obtenus en (b) pour déterminer la correspondance entre le profil de l'individu et le profil de la mère ainsi que le profil du père suspecté et établir ainsi sil le père suspecté est le père réel de l'individu.

5. Méthode pour déterminer la compatibilité d'un greffon ou d'une transfusion, basée sur le polymorphisme de la longueur des fragments de restriction d'ADN de HLA, caractérisée par les étapes suivantes:

(a) digérer l'ADN de HLA génomique du donneur du greffon ou de la transfusion et du receveur du greffon ou de la transfusion avec une endonucléase de restriction qui produit un produit de digestion polymorphe d'un locus ADN de HLA Classe II;

(b) soumettre chacun de produits de digestion de (a) au transfert ("blotting") génomique en utilisant une sonde ADNc marquée qui est complémentaire d'une séquence d'ADN de HLA Clase II impliquée dans le polymorphisme; et

(c) comparer les profils du transfert génomique obtenus en (b) pour déterminer la correspondance entre eux et établir ainsi si le HLA du donneur du greffon ou de la transfusion est compatible avec le HLA du receveur du greffon ou de la transfusion.

6. Méthode pour déterminer si un individu est sensible à une maladie ou en est atteint, caractérisée par les étapes suivantes:

(a) digérer l'ADN du HLA génomique de l'individu avec une endonucléase de restriction qui produit un profil de digestion polymorphe d'un locus HLA Classe II qui est associé à la maladie;

(b) soumettre le produit de digestion de (a) au transfert ("blotting") génomique en utilisant une sonde ADNc marquée qui est complémentaire de la séquence d'ADN dudit locus HLA Classe II;

(c) comparer le profil du transfert génomique obtenu en (b) avec un profil standard d'un individual atteint de ladite maladie, obtenu en utilisant ladite endonucléase de restriction et la même sonde ADNc marquée ou une sonde ayant la même spécificité.

7. L méthode de la Revendication 6 dans laquelle la maladie est le diabéte insulinodépendant.

22

FIG. 2

FIG. 1

FIG. 3

⊢——⊣ = 1kb
γ = Bgl II site
φ = Eco RV site
PM = polymorphic site
( ) = approximate boundry of HLADp34 coding sequence

6.8 kb   .99kb   9.2 kb   3.8 kb

3.8 kb

FIG. 4

FIG. 5
HIND III

| | FATHER |
| A/D | |
| E/F | MOTHER |
| D/E | CHILD 1 |
| D/F | CHILD 2 |
| D/E | CHILD 3 |
| A/E | CHILD 4 |
| | INDIVIDUAL X |
| | INDIVIDUAL Y |

FIG. 6
PVU II

| A/D | FATHER |
| E/F | MOTHER |
| D/E | CHILD 1 |
| D/F | CHILD 2 |
| A/E | CHILD 4 |
| D/E | CHILD 3 |

FIG. 7
BAM H1

| A/D | FATHER |
| E/F | MOTHER |
| D/E | CHILD 1 |
| D/F | CHILD 2 |
| D/E | CHILD 3 |
| A/E | CHILD 4 |
| | INDIVIDUAL X |

EP 0 084 796 B1

FIG. 8

4.5 kb →
4.2 kb →
3.8 kb →

1 FATHER   2 MOTHER   3 CHILD   4 CHILD   5 CHILD   6 CHILD   7 CHILD   8 CHILD

5

FIG. 9

CHILD 1 · CHILD 2 · CHILD 3 · MOTHER 4 · FATHER 5

4.5 kb →
4.2 kb →
3.8 kb →